(19) Europäisches Patentamt
European Patent Office
Office européen des brevets

(11) Veröffentlichungsnummer: **0 450 429 A1**

(12) **EUROPÄISCHE PATENTANMELDUNG**

(21) Anmeldenummer: **91104535.9**

(22) Anmeldetag: **22.03.91**

(51) Int. Cl.[5]: **C07D 211/26**, C07D 211/34, C07D 295/03, C07D 295/185, C07D 295/088, C07D 211/60, C07D 243/08, C07D 211/22, C07D 211/62, C07C 211/31, C07C 257/14

(30) Priorität: **02.04.90 DE 4010531**

(43) Veröffentlichungstag der Anmeldung: **09.10.91 Patentblatt 91/41**

(84) Benannte Vertragsstaaten: **AT BE CH DE DK ES FR GB GR IT LI LU NL SE**

(71) Anmelder: **Dr. Karl Thomae GmbH**
**Postfach 1755**
**W-7950 Biberach 1(DE)**

(72) Erfinder: **Psiorz, Manfred, Dr. Dipl.-Chem.**
**Matthias-Grünewald-Strasse 1**
**W-6507 Ingelheim/Rhein(DE)**
Erfinder: **Trach, Volker, Dr.**
**Probststrasse 7**
**W-7950 Biberach 1(DE)**

(54) **Cyclophane, diese Verbindungen enthaltende Arzneimittel und Verfahren zu ihrer Herstellung.**

(57) Die vorliegende Erfindung betrifft Cyclophane der allgemeinen Formel

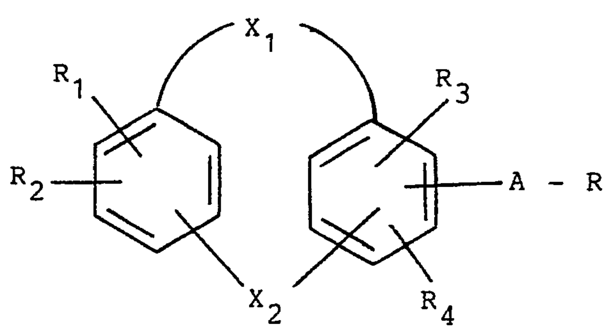

, (I)

in der
$X_1$, $X_2$, A, R und $R_1$ bis $R_4$ wie im Anspruch 1 definiert sind, deren Enantiomere, deren Diastereomere, deren Additionssalze, insbesondere für die pharmazeutische Anwendung deren physiologisch verträgliche Additionssalze, welche wertvolle Eigenschaften aufweisen, insbesondere wertvolle pharmakologische Eigenschaften wie eine blutdrucksenkende und coronardilatatierende sowie eine milde herzfrequenzsenkende Wirkung, diese Verbindungen enthaltende Arzneimittel und Verfahren zu ihrer Herstellung.

EP 0 450 429 A1

Die vorliegende Erfindung betrifft Cyclophane der allgemeinen Formel

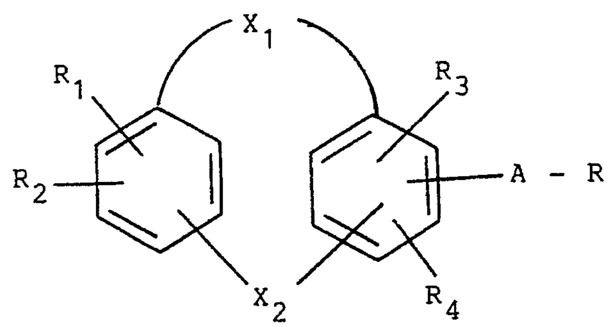

, (I)

deren Enantiomere, deren Diastereomere, deren Additionssalze, insbesondere für die pharmazeutische Anwendung deren physiologisch verträgliche Additionssalze, diese Verbindungen enthaltende Arzneimittel und Verfahren zu ihrer Herstellung.

Die neuen Verbindungen weisen wertvolle Eigenschaften auf, insbesondere wertvolle pharmakologische Eigenschaften wie eine blutdrucksenkende und coronardilatatierende sowie eine milde herzfrequenzsenkende Wirkung.

In der obigen allgemeinen Formel I bedeutet

$X_1$ und $X_2$, die gleich oder verschieden sein können, eine geradkettige Alkylen- oder Alkenylengruppe mit jeweils 2 bis 4 Kohlenstoffatomen,

A eine geradkettige Alkylengruppe mit 1 bis 6 Kohlenstoffatomen, eine $-Y_1-A_1-$ oder $-Y_2-A_2-$Gruppe, in denen

$Y_1$ ein Sauerstoffatom, eine Sulfenyl-, Sulfinyl- oder Sulfonylgruppe und

$A_1$ eine geradkettige Alkylengruppe mit 2 bis 4 Kohlenstoffatomen,

$Y_2$ eine Ethenylen- oder Ethinylengruppe und

$A_2$ eine geradkettige Alkylengruppe mit 1 bis 3 Kohlenstoffatomen bedeuten,

wobei die Reste $A_1$ und $A_2$ mit dem Rest R verknüpft sind und das Kohlenstoffgerüst der Gruppen A, $A_1$ und $A_2$ zusätzlichdurch eine oder zwei Methylgruppen substituiert sein kann,

$R_1$, $R_2$, $R_3$ und $R_4$, die gleich oder verschieden sein können, ein Wasserstoff- oder Halogenatom, eine Alkyl-, Hydroxy-, Alkoxy- oder Alkylsulfonyloxygruppe mit jeweils 1 bis 3 Kohlenstoffatomen im Alkylteil und

R eine Cyanogruppe oder eine Gruppe der Formeln

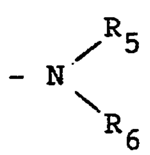

oder

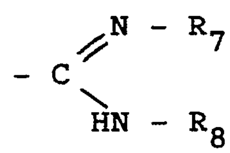

,

in denen

$R_5$ ein Wasserstoffatom, eine Alkylgruppe mit 1 bis 10 Kohlenstoffatomen, eine Cycloalkylgruppe mit 5 bis 7 Kohlenstoffatomen, eine bi- oder tricyclische Alkylgruppe mit 6 bis 15 Kohlenstoffatomen, wobei jeder der vorstehend erwähnten cyclischen Reste durch eine oder zwei Alkylgruppen mit jeweils 1 bis 3 Kohlenstoffatomen substituiert sein kann, oder eine Phenylalkylgruppe, in der der Alkylteil 1 bis 3 Kohlenstoffatome enthalten kann und der Phenylkern durch ein Halogenatom, durch eine Alkyl- oder Alkoxygruppe mit jeweils 1 bis 3 Kohlenstoffatomen mono-, di- oder trisubstituiert sein kann,

$R_6$ ein Wasserstoffatom oder eine Alkylgruppe mit 1 bis 5 Kohlenstoffatomen oder

$R_5$ und $R_6$ zusammen mit dem dazwischenliegenden Stickstoffatom eine Pyrrolidino-, Piperidino-, Hexamethylenimino- oder Morpholinogruppe, wobei

i) in einer vorstehend bei der Definition der Reste $R_5$ und $R_6$ erwähnten Piperidino- oder Hexamethyleniminogruppe eine Methylengruppe in 4-Stellung durch eine Imino-, Alkylimino-, Alkoxycarbonylimino-, Alkanoylimino- oder Phenylalkyliminogruppe oder durch eine in 2- oder 3-Stellung durch eine Phenoxygruppe substituierte Alkyliminogruppe mit 2 oder 3 Kohlenstoffatomen oder durch eine Phenylalkenyliminogruppe, in welcher der Alkenylteil 2 oder 3 Kohlenstoffatome enthalten kann, ersetzt sein kann oder

ii) eine vorstehend bei der Definition der Reste $R_5$ und $R_6$ erwähnte Piperidinogruppe durch eine Hydroxy-, Alkoxy-, Carboxy-, Alkoxycarbonyl, Aminocarbonyl-, Alkylaminocarbonyl- oder Dialkylaminocarbonylgruppe oder durch eine Alkylgruppe mit 1 bis 5 Kohlenstoffatomen, welche durch eine Hydroxy-, Alkoxy-, Alkanoyloxy-, Cyano-, Carboxy-, Alkoxycarbonyl-, Aminocarbonyl-, Alkylaminocarbonyl-, Dialkylaminocarbonyl-, Amino-, Alkylamino-, Dialkylamino-, Alkanoylamino-, Alkylsulfonylamino-, N-Alkanoyl-alkylamino- oder N-Alkylsulfonyl-alkylaminogruppe substituiert sein kann, wobei, soweit nichts anderes erwähnt wurde, die unter i) und ii) vorstehend erwähnten Alkyl-, Alkoxy- und Alkanoylteile jeweils 1 bis 3 Kohlenstoffatome enthalten können und die vorstehend erwähnten Phenylkerne jeweils durch eine Nitro- oder Trifluormethylgruppe monosubstituiert oder durch Halogenatome, Alkylgruppen mit 1 bis 3 Kohlenstoffatomen oder Alkyloxygruppen mit 1 bis 3 Kohlenstoffatomen mono-, di- oder trisubstituiert und die Substituenten gleich oder verschieden sein können, und

$R_7$ und $R_8$, die gleich oder verschieden sein können, Wasserstoffatome oder Alkylgruppen mit 1 bis 3 Kohlenstoffatomen bedeuten.

Bevorzugte Verbindungen sind jedoch die [2.2]Paracyclophane der allgemeinen Formel

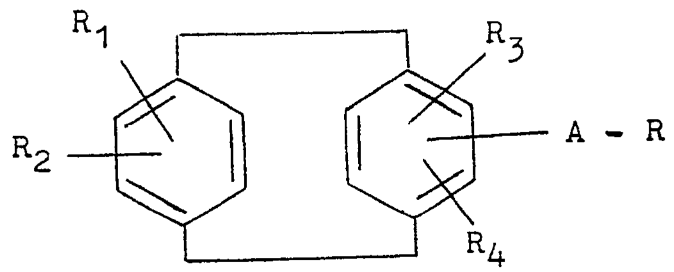

,(Ia)

in der

A eine geradkettige Alkylengruppe mit 1 bis 5 Kohlenstoffatomen, eine $-Y_1-A_1-$ oder $-Y_2-A_2-$Gruppe, in denen

$Y_1$ ein Sauerstoffatom und

$A_1$ eine geradkettige Alkylengruppe mit 2 oder 3 Kohlenstoffatomen,

$Y_2$ eine Ethenylen- oder Ethinylengruppe und

$A_2$ eine Methylengruppe bedeuten,

wobei die Reste $A_1$ und $A_2$ mit dem Rest R verknüpft sind,

einer der Reste $R_1$, $R_2$, $R_3$ oder $R_4$ ein Wasserstoff-, Fluor-, Chlor- oder Bromatom, eine Methyl-, Hydroxy-, Methoxy- oder Methansulfonyloxygruppe und

die übrigen der Reste $R_1$, $R_2$, $R_3$ oder $R_4$ jeweils ein Wasserstoffatom oder eine Methylgruppe, und

R eine Cyanogruppe oder eine Gruppe der Formeln

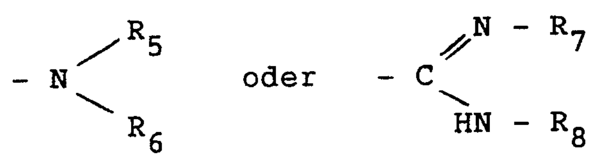

darstellen, in denen

$R_5$ ein Wasserstoffatom, eine gegebenenfalls durch eine Phenyl- oder Dimethoxyphenylgruppe substituierte Alkylgruppe mit 1 bis 3 Kohlenstoffatomen, eine Alkylgruppe mit 4 bis 8 Kohlenstoffatomen, eine Cycloalkylgruppe mit 5 bis 7 Kohlenstoffatomen oder eine bi- oder tricyclische Alkylgruppe mit 7 bis 12 Kohlenstoffatomen, wobei jeder der vorstehend erwähnten cyclischen Reste durch eine oder zwei Methylgruppen substituiert sein kann,

$R_6$ ein Wasserstoffatom oder eine Alkylgruppe mit 1 bis 3 Kohlenstoffatomen oder

$R_5$ und $R_6$ zusammen mit dem dazwischenliegenden Stickstoffatom eine Pyrrolidino-, Piperidino-, Hexamethylenimino- oder Morpholinogruppe, wobei

i) in einer vorstehend bei der Definition der Reste $R_5$ und $R_6$ erwähnten Piperidino- oder Hexamethyleni-

minogruppe eine Methylengruppe in 4-Stellung durch eine Imino-, Methylimino-, Ethoxycarbonylimino-, Acetylimino-, Phenylethylimino-, Phenoxyethylimino-oder Phenylallyliminogruppe ersetzt sein kann oder
ii) eine vorstehend bei der Definition der Reste $R_5$ und $R_6$ erwähnte Piperidinogruppe durch eine Carboxy-, Ethoxycarbonyl-, Aminocarbonyl-, Methylaminocarbonyl- oder Dimethylaminocarbonylgruppe oder durch eine durch eine Hydroxy-, Acetoxy-, Cyano-, Carboxy-, Ethoxycarbonyl-, Aminocarbonyl-, Methylaminocarbonyl-, Dimethylaminocarbonyl-, Amino-, Methylamino-, Dimethylamino-, Diethylamino-, Acetylamino-, N-Acetyl-methylamino-, Methansulfonylamino- oder N-Methansulfonyl-methylaminogruppe substituierte Methylgruppe oder durch eine in 2-Stellung durch eine Hydroxy-, Carboxy- oder Ethoxycarbonylgruppe substituierte Ethylgruppe oder durch eine in 2-, 3- oder 4-Stellung durch eine Dimethylaminocarbonylgruppe substituierte Alkylgruppe mit 2 bis 4 Kohlenstoffatomen substituiert sein kann, wobei, soweit nichts anderes erwähnt wurde, die unter i) und ii) vorstehend erwähnten Phenylkerne jeweils durch eine Nitro- oder Trifluormethylgruppe monosubstituiert oder durch Methyl- oder Methoxygruppen mono-, di- oder trisubstituiert und die Substituenten gleich oder verschieden sein können, und
$R_7$ und $R_8$, die gleich oder verschieden sein können, Wasserstoffatome oder Methylgruppen bedeuten, deren Enantiomere, deren Diastereomere, deren Additionssalze, insbesondere für die pharmazeutische Anwendung deren physiologisch verträglichen Additionssalze.

Besonders bevorzugte Verbindungen der allgemeinen Formel Ia sind diejenigen, in denen
in der
A eine geradkettige Alkylengruppe mit 2 bis 4 Kohlenstoffatomen, eine $-Y_1-A_1-$ oder $-Y_2-A_2-$Gruppe, in denen
$Y_1$ ein Sauerstoffatom und
$A_1$ eine geradkettige Alkylengruppe mit 2 oder 3 Kohlenstoffatomen,
$Y_2$ eine Ethenylen- oder Ethinylengruppe und
$A_2$ eine Methylengruppe bedeuten,
wobei die Reste $A_1$ und $A_2$ mit dem Rest R verknüpft sind,
$R_1$, $R_2$, $R_3$ und $R_4$ jeweils ein Wasserstoffatom und
R eine Gruppe der Formeln

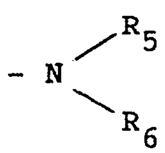

oder

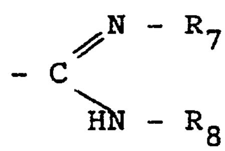

darstellen, in denen
$R_5$ ein Wasserstoffatom, eine gegebenenfalls durch eine Phenyl- oder Dimethoxyphenylgruppe substituierte Alkylgruppe mit 1 bis 3 Kohlenstoffatomen, eine Alkylgruppe mit 4 bis 8 Kohlenstoffatomen, eine Cycloalkylgruppe mit 5 bis 7 Kohlenstoffatomen oder eine bi- oder tricyclische Alkylgruppe mit 7 bis 12 Kohlenstoffatomen, wobei jeder der vorstehend erwähnten cyclischen Reste durch eine oder zwei Methylgruppen substituiert sein kann,
$R_6$ ein Wasserstoffatom oder eine Alkylgruppe mit 1 bis 3 Kohlenstoffatomen oder
$R_5$ und $R_6$ zusammen mit dem dazwischenliegenden Stickstoffatom eine Piperidinogruppe, wobei
i) in einer vorstehend bei der Definition der Reste $R_5$ und $R_6$ erwähnten Piperidinogruppe eine Methylengruppe in 4-Stellung durch eine Imino-, Methylimino-, Ethoxycarbonylimino-, Acetylimino-, Phenylethylimino-, Phenoxyethylimino- oder Phenylallyliminogruppe ersetzt sein kann oder
ii) eine vorstehend bei der Definition der Reste $R_5$ und $R_6$ erwähnte Piperidinogruppe in 3- oder 4-Stellung durch eine Carboxy-, Ethoxycarbonyl-, Aminocarbonyl-, Methylaminocarbonyl- oder Dimethylaminocarbonylgruppe oder durch eine durch eine Hydroxy-, Acetoxy-, Cyano-, Carboxy-, Ethoxycarbonyl-, Aminocarbonyl-, Methylaminocarbonyl-, Dimethylaminocarbonyl-, Amino-, Methylamino-, Dimethylamino-, Diethylamino-, Acetylamino-, N-Acetyl-methylamino-, Methansulfonylamino- oder N-Methansulfonyl-methylaminogruppe substituierte Methylgruppe oder durch eine in 2-Stellung durch eine Hydroxy-, Carboxy- oder Ethoxycarbonylgruppe substituierte Ethylgruppe oder durch eine in 2-, 3- oder 4-Stellung durch eine Dimethylaminocarbonylgruppe substituierte Alkylgruppe mit 2 bis 4 Kohlenstoffatomen substituiert sein kann, wobei, soweit nichts anderes erwähnt wurde, die unter i) und ii) vorstehend erwähnten Phenylkerne jeweils durch eine Methyl-, Nitro- oder Trifluormethylgruppe monosubstituiert oder durch Methoxygruppen mono-, di- oder trisubstituiert sein können, und
$R_7$ und $R_8$ jeweils ein Wasserstoffatom oder eine Methylgruppe bedeuten, deren Enantiomere, deren

Diastereomere, deren Additionssalze, insbesondere für die pharmazeutische Anwendung deren physiologisch verträglichen Additionssalze.

Erfindungsgemäß erhält man die neuen Verbindungen nach folgenden Verfahren:

a) Umsetzung einer Verbindung der allgemeinen Formel

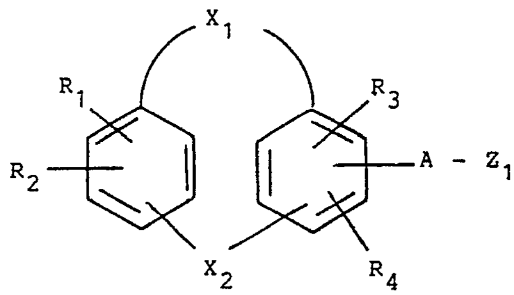

,(II)

in der

$R_1$ bis $R_4$, $X_1$, $X_2$ und A wie eingangs definiert sind und

$Z_1$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt, mit einem Amin der allgemeinen Formel

H - R ,(III)

in der

R wie eingangs definiert ist, oder mit dessen Alkalisalz und, zur Herstellung einer entsprechenden Amidinoverbindung der allgemeinen Formel I, anschließende Überführung einer so erhaltenen Cyanoverbindung der allgemeinen Formel I über ihren Iminoester mit einem Amin der allgemeinen Formel

$R_7 - NH_2$ ,(IV)

in der

$R_7$ wie eingangs definiert ist.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und der Siedetemperatur des Reaktionsgemisches, durchgeführt.

Die anschließende Überführung einer so erhaltenen Cyanoverbindung in eine entsprechende Amidinoverbindung wird vorzugsweise in einem Lösungsmittel wie Diethylether, Tetrahydrofuran, Dioxan oder Methanol/Diethylether bei Temperaturen zwischen 0 und 20°C durchgeführt, wobei gegebenenfalls unter Schutzgas, z.B. unter Stickstoff, zuerst mittels einer alkoholischen Salzsäure, welche beispielsweise durch Einleiten von Chlorwasserstoff in absolutem Methanol/Diethylether erhalten wird, der entsprechende Iminoester hergestellt wird, welcher anschließend mit einem Amin der Formel IV in einem Lösungsmittel wie Methanol oder Diethylether/$R_7$-$NH_2$ bei 0°C in das entsprechende Amidin übergeführt wird.

b) Zur Herstellung einer Verbindung der allgemeinen Formel I, in der R keine Cyano-, Amidino- oder Guanidinogruppe und $Y_1$ keine Sulfinyl- oder Sulfonylgruppe darstellen sowie der Rest R keine Carbonylfunktion enthält:

Reduktion einer Verbindung der allgemeinen Formel

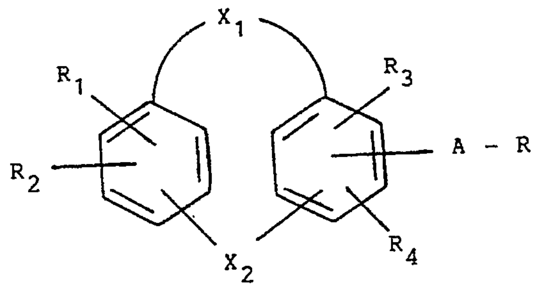

,(V)

in der

R und A wie eingangs definiert sind, wobei jedoch in A, $A_1$ oder $A_2$ eine zum Rest R benachbarte $-CH_2-$Gruppe durch eine -CO-Gruppe oder

eine benachbarte $-CH_2-CH_2-$Gruppe durch eine -CO-CO-Gruppe ersetzt sein muß, mit einem komplexen Metallhydrid.

Die Reduktion wird vorzugsweise in einem geeigneten Lösungsmittel wie Diethylether oder Tetrahydrofuran und gegebenenfalls unter Schutzgas, z.B. unter Stickstoff, in Gegenwart eines Metallhydrids wie Lithiumaluminiumhydrid, Diboran oder Boran/Dimethylsulfid zwischen O und 80° C, vorzugsweise jedoch bei der Siedetemperatur des Reaktionsgemisches, durchgeführt.

Bei der Reduktion wird eine im Rest R vorhandene Carbonylfunktion gleichzeitig mitreduziert.

Bei den vorstehend beschriebenen Umsetzungen können gegebenenfalls vorhandene reaktive Gruppen wie Hydroxy-, Amino-, Imino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100° C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50° C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der allgemeinen Formel I, in der R eine Alkoxycarbonylgruppe enthält, so läßt sich diese mittels Hydrolyse in eine entsprechende Hydroxycarbonylverbindung der allgemeinen Formel I überführen, wobei gleichzeitig eine im Reaktionsgemisch entstehende Hydroxycarbonylimino- oder Hydroxycarbonylaminoverbindung der allgemeinen Formel I beim Erhitzen zu der entsprechenden Imino- oder Aminoverbindung der allgemeinen Formel I decarboxyliert wird, oder

eine Verbindung der allgemeinen Formel I, in der R eine Aminocarbonylgruppe enthält, so läßt sich diese mittels Dehydratisierung in eine entsprechende Cyanoverbindung der allgemeinen Formel I überführen oder eine Verbindung der allgemeinen Formel I, in der R eine Imino-, Amino- oder Alkylaminogruppe enthält, so läßt sich diese mittels Acylierung in eine entsprechende Alkanoylimino-, Alkanoylamino-, N-Alkanoyl-alkylamino-, Alkylsulfonylimino-, Alkylsulfonylamino- oder N-Alkylsulfonyl-alkylaminoverbindung der allgemeinen Formel I überführen oder

eine Verbindung der allgemeinen Formel I, in der $R_5$ und $R_6$ jeweils ein Wasserstoffatom darstellen, so läßt sich diese mittels Umamidinierung in eine entsprechende Amidinoverbindung der allgemeinen Formel I überführen.

Die nachträgliche Hydrolyse erfolgt vorzugsweise hydrolytisch in einem wässrigen Lösungsmittel, z.B. in Wasser, Ethanol/Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100° C, vorzugsweise bei der Siedetemperatur des Reaktionsgemisches.

Die nachträgliche Dehydratisierung wird mit einem wasserentziehenden Mittel wie Phosphorpentoxid, Phosphoroxychlorid, Schwefelsäure oder p-Toluolsulfonsäurechlorid gegebenenfalls in einem Lösungsmittel

wie Methylenchlorid oder Pyridin bei Temperaturen zwischen 0 und 100° C, vorzugsweise bei Temperaturen zwischen 20 und 80° C, durchgeführt.

Die nachträgliche Acylierung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100° C, vorzugsweise jedoch bei Temperaturen zwischen -10 und 80° C, durchgeführt.

Die nachträgliche Umamidinierung wird vorzugsweise in einem Lösungsmittel wie Dimethylformamid, Dimethylacetamid oder Dimethylformamid/Wasser und zweckmäßigerweise in Gegenwart einer tertiären organischen Base wie Triethylamin oder Pyridin in Gegenwart eines Amidins wie 3,5-Dimethylpyrazol-1-carbonsäureamidin bei Temperaturen zwischen 0 und 50° C, vorzugsweise bei Raumtemperatur, durchgeführt.

Ferner können die erfindungsgemäß erhaltenen Verbindungen der allgemeinen Formel I in ihre Enantiomeren und, sofern der Rest R mindestens ein weiteres chirales Zentrum enthält, in ihre Diastereomeren nach bekannten Methoden aufgetrennt werden, z.B. durch Säulenchromatographie an einer chiralen Phase oder durch Kristallisation mit optisch aktiven Säuren wie mit D- oder L-Monomethylweinsäure, D- oder L-Diacetylweinsäure, D- oder L-Weinsäure, D- oder L-Milchsäure oder D- oder L-Camphersäure.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis V sind literaturbekannt bzw. lassen sich nach an sich bekannten Methoden herstellen.

Eine Ausgangsverbindung der Formel II erhält man durch Umsetzung eines entsprechenden Hydroxyalkyl-paracyclophans mit einem Halogenierungsmittel und eine Ausgangsverbindung der Formel V durch Amidierung eines entsprechenden Hydroxycarbonylalkyl-paracyclophans.

Eine optisch aktive Ausgangsverbindung der allgemeinen Formel II oder V erhält man ausgehend von einem entsprechend optisch aktiven 4-Carboxy-paracyclophan (siehe beispielsweise H. Falck et al. in Tetrahedron 26, 511-527 (1970)) durch Überführung der Carboxygruppe in einen Rest $-A-Z_1$ oder -A-R nach üblichen Methoden, beispielsweise durch Reduktion und anschließende Halogenierung oder durch Oxidation des so erhaltenen Alkohols, anschließende Esterkondensation, Hydrierung und Überführung in das entsprechende Amid.

Wie bereits eingangs erwähnt weisen die neuen Verbindungen der Formel I wertvolle Eigenschaften auf. Die Verbindungen der Formel I, in der R eine Gruppe der Formeln

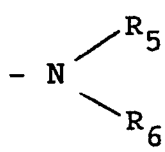

oder

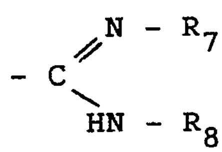

darstellen, wobei

$R_5$ bis $R_8$ wie eingangs definiert sind, deren Enantiomeren, deren Diastereomeren und deren physiologisch verträgliche Additionssalze weisen wertvolle pharmakologische Eigenschaften auf, insbesondere eine blutdrucksenkende und coronardilatatierende sowie eine milde herzfrequenzsenkende Wirkung.

Ferner stellen die Verbindungen der Formel I, in der R eine Cyanogruppe darstellt, wertvolle Zwischenprodukte zur Herstellung der entsprechenden Amino-, Amidino- oder Guanidinoverbindung der Formel I dar.

Beispielsweise wurden die Verbindungen

A = 4-(3-(Piperidin-1-yl)prop-1-yl)-[2.2]-paracyclophan-hydrochlorid,
B = 4-(3-(3-(N-Acetyl-methylaminomethyl)piperidin-1-yl)prop-1-yl)-[2.2]paracyclophan-hydrochlorid,
C = 4-Methyl-7-(3-(3-(N-acetyl-methylaminomethyl)-piperidin-1-yl)prop-1-yl)[2.2]paracyclophan-oxalat,
D = 4-(3-(3-(3-Dimethylamino-3-oxo-prop-1-yl)piperidin-1-yl)prop-1-yl)-[2.2]paracyclophan-oxalat und
E = 4-(2-(Piperidin-1-yl)ethoxy-[2.2]paracyclophan-oxalat

auf ihre biologischen Eigenschaften wie folgt untersucht:

Wirkung auf den Blutdruck an Ratten:

Die Wirkung der zu untersuchenden Substanzen auf den Blutdruck wurde pro Dosis an 2 Ratten mit einem durchschnittlichen Gewicht von 350-400 g untersucht. Hierzu wurden die Ratten mit Pentobarbital (50 mg/kg i.p.) narkotisiert. Die zu untersuchenden Substanzen wurden in wäßriger Lösung in die Vena femoralis injiziert (0,1 ml/100 g).

Der Blutdruck wurde über in eine A. carotis eingebundene Kanüle gemessen und die Herzfrequenz aus dem Blutdrucksignal abgeleitet.

Die nachfolgende Tabelle enthält die gefundenen Werte:

| Substanz | Dosis [mg/kg i.v.] | max. Blutdruck-senkung in % | Herzfrequenz-senkung in % |
|---|---|---|---|
| A | 1.0 | - 27.8 | - 10.4 |
| B | 1.0 | - 43.1 | - 18.0 |
| C | 1.0 | - 32.4 | - 14.1 |
| D | 1.0 | - 48.0 | - 17.9 |
| E | 1.0 | - 24.2 | - 24.8 |

Bei einer Dosis von 30 mg/kg i.v. der vorstehend erwähnten Verbindungen A bis E konnten keine toxischen Nebenwirkungen beobachtet werden. Die neuen Verbindungen sind daher in therapeutischen Dosen gut verträglich.

Aufgrund ihrer pharmakologischen Eigenschaften eignen sich die neuen Verbindungen und deren physiologisch verträgliche Additionssalze zur Behandlung der Hypertonie sowie zur Prophylaxe und Therapie ischämischer Herzerkrankungen.

Die zur Erzielung einer entsprechenden Wirkung erforderliche Dosierung beträgt zweckmäßigerweise bei intravenöser Gabe 2,5 bis 20 mg, vorzugsweise 5 bis 10 mg, und bei oraler Gabe 40 bis 120 mg, vorzugsweise 60 bis 100 mg, jeweils 1 bis 2 x täglich. Hierzu lassen sich die erfindungsgemäß hergestellten Verbindungen der Formel, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zapfchen einarbeiten.

Die nachfolgenden Beispiele sollen die Erfindung näher erläutern:

Vorbemerkung:

Die Nomenklatur und Numerierung des Cyclophansystems erfolgte analog in der von Vögtle et al. in Tetrahedron 26, 5847 (1970) vorgeschlagenen Weise.

Beispiel A

4-(3-Hydroxyprop-1-yl)-[2.2]paracyclophan

Zu 9,12 g (0,23 Mol) Lithiumaluminiumhydrid in 300 ml absolutem Tetrahydrofuran werden bei 35 °C unter Eiskühlung 44,86 g (0,16 Mol) 4-(2-Carboxyeth-1-yl)-[2.2]paracyclophan, gelöst in 300 ml absolutem Tetrahydrofuran, getropft. Danach wird eine Stunde bei Raumtemperatur gerührt, anschließend unter Eiswasserkühlung mit 9,1 ml Wasser, 9,1 ml 15%iger Natronlauge und 27 ml Wasser versetzt. Der Niederschlag wird abgesaugt, mit Tetrahydrofuran gewaschen und das Filtrat im Vakuum eingeengt.
Ausbeute: 40,1 g (94 % der Theorie),
Schmelzpunkt: 78-80 °C

Beispiel B

4-(3-Bromprop-1-yl)-[2.2]paracylophan

32,0 g (0,12 Mol) 4-(3-Hydroxyprop-1-yl)-[2.2]paracylophan und 36,7 g (0,14 Mol) Triphenylphosphin werden in 650 ml Methylenchlorid gelöst und auf 5 °C gekühlt. Anschließend tropft man unter Eiswasserkühlung 46,4 g (0,14 Mol) Tetrabromkohlenstoff, gelöst in 70 ml Methylenchlorid, zu. Nach 1-stündigem Rühren bei Raumtemperatur wird das Reaktionsgemisch in 350 ml Wasser gegossen. Die organische Phase wird abgetrennt, über Magnesiumsulfat getrocknet und im Vakuum eingeengt. Der Rückstand wird säulenchromatographisch über Kieselgel (Korngröße:
30-60 µm, Elutionsmittel: Cyclohexan) gereinigt.
Ausbeute: 28,3 g (72 % der Theorie),
Schmelzpunkt: 90-92 °C

Beispiel C

4-(5-(4-Methylpiperazin-1-yl)-5-oxo-pent-1-yl)-[2.2]paracyclophan

1,23 g (0,004 Mol) 4-(4-Carboxybut-1-yl)-[2.2]paracyclophan werden in 20 ml Essigester gelöst, mit 0,65 g (0,004 Mol) N,N'-Carbonyldiimidazol versetzt und 90 Minuten bei 60 °C gerührt. Zu dieser Lösung werden 0,44 ml (0,004 Mol) N-Methylpiperazin gegeben. Danach wird eine Stunde bei 60 °C gerührt, anschließend das abgekühlte Reaktionsgemisch 2 x mit 8%iger Natronlauge extrahiert. Die organische Phase wird über Magnesiumsulfat getrocknet und im Vakuum eingedampft.
Ausbeute: 1,28 g (82 % der Theorie),
$R_f$-Wert: 0,5 (Aluminiumoxid, neutral; Laufmittel: 3 % Ethanol in Methylenchlorid)

Beispiel D

4-(5-(2-(Diethylaminomethyl)piperidin-1-yl)-5-oxo-pent-1-yl)-[2.2]paracyclophan

1,23 g (0,004 Mol) 4-(4-Carboxybut-1-yl)-[2.2]paracyclophan werden in 30 ml Methylenchlorid gelöst, mit 1,2 ml (0,0165 Mol) Thionylchlorid versetzt und 2 Stunden unter Rückfluß erhitzt. Nach Einengen im Vakuum wird der erhaltene Rückstand in 15 ml Methylenchlorid gelöst und zu einer Lösung von 0,68 g (0,004 Mol) 2-(Diethylaminomethyl)piperidin und 0,7 ml (0,005 Mol) Triethylamin in 20 ml Methylenchlorid getropft. Danach wird eine Stunde bei Raumtemperatur gerührt, anschließend 2 mal mit 8%iger Natronlauge und einmal mit Wasser extrahiert. Die organische Phase wird über Magnesiumsulfat getrocknet und im Vakuum eingedampft.
Ausbeute: 1,80 g (98 % der Theorie),
$R_f$-Wert: 0,55 (Aluminiumoxid, neutral; Laufmittel: 3 % Ethanol in Methylenchlorid)

Beispiel E

4-(3-(N-(3-Methylaminoprop-1-yl)methylamino)prop-1-yl)-[2.2]paracyclophan

a) 4-(3-(N-(2-Cyanoeth-1-yl)methylamino)prop-1-yl)-[2.2]paracyclophan

Ein Gemisch von 1,40 g (0,005 Mol) 4-(3-Methylaminoprop-1-yl)-[2.2]paracyclophan und 0,41 ml (0,00625 Mol) Acrylnitril in 15 ml Methanol wird 3 Stunden bei Raumtemperatur gerührt. Das Lösungsmittel wird im Vakuum eingedampft und der erhaltene Rückstand über Aluminiumoxid (neutral, Aktivität II-III) mit Methylenchlorid/Cyclohexan (3/1) gereinigt.
Ausbeute: 1,4 g (84 % der Theorie),
$R_f$-Wert: 0,4 (Kieselgel, Laufmittel: 3 % Ethanol in Methylenchlorid)

b) 4-(3-(N-(3-Aminoprop-1-yl)methylamino)prop-1-yl)-[2.2]paracyclophan

1,3 g (0,0039 Mol) 4-(3-(N-(2-Cyanoeth-1-yl)methylamino)prop-1-yl)-[2.2]paracyclophan werden in 25 ml methanolischem Ammoniak in Anwesenheit von 0,4 g Raney-Nickel 5 Stunden bei 50 °C und 4 bar Wasserstoff hydriert. Anschließend wird der Katalysator abgesaugt und das Lösungsmittel im Vakuum abdestilliert.
Ausbeute: 1,28 g (98 % der Theorie),
$R_f$-Wert: 0,1 (Aluminiumoxid, Laufmittel: 5 % Ethanol in Methylenchlorid)

c) 4-(3-(N-(3-Formylaminoprop-1-yl)methylamino)prop-1-yl)-[2.2]paracyclophan

Ein Gemisch von 1,28 g (0,0038 Mol) 4-(3-(N-(3-Aminoprop-1-yl)methylamino)prop-1-yl)-[2.2]-paracyclophan und 0,17 ml (0,0045 Mol) Ameisensäure in 20 ml Toluol wird am Wasserabscheider 3 Stunden unter Rückfluß erhitzt. Das abgekühlte Reaktionsgemisch wird mit 2 molarer Natronlauge extrahiert. Die organische Phase wird abgetrennt, über Magnesiumsulfat getrocknet, im Vakuum eingedampft und über Aluminiumoxid (neutral, Aktivität II-III) mit Methylenchlorid und anschließend mit steigenden Anteilen von Ethanol (bis 2 %) gereinigt.
Ausbeute: 0,50 g (36 % der Theorie),
$R_f$-Wert: 0,3 (Aluminiumoxid, Laufmittel: 3 % Ethanol in Methylenchlorid)

d) 4-(3-(N-(3-Methylaminoprop-1-yl)methylamino)prop-1-yl)-[2.2]paracyclophan

Hergestellt durch Reduktion von 4-(3-(N-(3-Formylaminoprop-1-yl)methylamino)prop-1-yl)-[2.2]-paracyclophan und Lithiumaluminiumhydrid in Tetrahydrofuran.
Ausbeute: 97 % der Theorie,
$R_f$-Wert: 0,15 (Aluminiumoxid, Laufmittel: 5 % Ethanol in Methylenchlorid)

Beispiel F

4-(2-Chlorethoxy)-[2.2]paracyclophan

0,9 g (0,004 Mol) 4-Hydroxy-[2.2]paracyclophan werden in 40 ml Aceton gelöst, mit 4,1 g (0,03 Mol) Kaliumcarbonat versetzt und 30 Minuten bei 40 °C gerührt. Zu dieser Suspension werden 2,5 ml (0,03 Mol) 1-Brom-2-chlorethan zugetropft. Nach 17-stündigem Erhitzen am Rückfluß wird das abgekühlte Reaktionsgemisch filtriert und das Filtrat im Vakuum eingedampft. Der Rückstand wird säulenchromatographisch über Aluminiumoxid (neutral, Aktivität II-III) mit einem Petrolether/Essigester (10/1) gereinigt.
Ausbeute: 0,75 g (65 % der Theorie),
Schmelzpunkt: 104-105 °C

Beispiel G

4-(3-Cyanoprop-1-yl)-[2.2]paracyclophan

Zu einer Lösung von 0,55 g (0,011 Mol) Natriumcyanid in 30 ml Dimethylsulfoxid werden 2,5 g (0,008 Mol) 4-(3-Bromprop-1-yl)-[2.2]paracyclophan zugegeben. Nach 2-stündigem Rühren bei 90 °C wird auf Eiswasser gegossen. Die wässrige Lösung wird 3 x mit Essigester extrahiert. Die vereinigten organischen Phasen werden über Magnesiumsulfat getrocknet und im Vakuum eingedampft. Der Rückstand wird

säulenchromatographisch über Aluminiumoxid (neutral, Aktivität II-III) mit Methylenchlorid/Cyclohexan (1/1) gereinigt.
Ausbeute: 1,7 g (81 % der Theorie),
Schmelzpunkt: 81 °C

Beispiel 1

4-(3-(3-(Ethoxycarbonylmethyl)piperidin-1-yl)prop-1-yl)-[2.2]paracyclophan-tartrat

Ein Gemisch von 0,85 g (0,005 Mol) (Piperidin-3-yl)essigester, 0,7 ml (0,005 Mol) Triethylamin und 1,65 g (0,005 Mol) 4-(3-Bromprop-1-yl)-[2.2]paracyclophan wird 2 Stunden unter Rückfluß erhitzt. Die anfängliche Suspension geht in eine klare Lösung über und beginnt nach etwa 20 Minuten gallertartig zu werden. Das abgekühlte Reaktionsgemisch wird in einem Gemisch aus Wasser und Essigester gelöst. Die organische Phase wird abgetrennt, mit Wasser gewaschen, über Magnesiumsulfat getrocknet, im Vakuum eingedampft und über 150 g Aluminiumoxid (neutral, Aktivität II-III) mit Petrolether/Essigester (9/1) gereinigt. Aus einer Lösung in Aceton/Ether wird mit Weinsäure das Tartrat gefällt.
Ausbeute: 1,80 g (63 % der Theorie),
Schmelzpunkt: 68-70 °C (Zers.)

| | | | | | | |
|---|---|---|---|---|---|---|
| Ber.: | C | 67,47 | H | 7,61 | N | 2,46 |
| Gef.: | | 67,35 | | 7,63 | | 2,36 |

Beispiel 2

4-(5-(2-(Diethylaminomethyl)piperidin-1-yl)pent-1-yl)-[2.2]paracyclophan-dihydrochlorid

Zu 0,38 g (0,01 Mol) Lithiumaluminiumhydrid in 20 ml absolutem Tetrahydrofuran werden 1,13 g (0,0029 Mol) 4-(5-(2-(Diethylaminomethyl)piperidin-1-yl)-5-oxo-pent-1-yl)-[2.2]paracyclophan, gelöst in 15 ml absolutem Tetrahydrofuran, getropft. Danach wird 90 Minuten bei Raumtemperatur gerührt und anschließend unter Eiswasserkühlung mit 0,38 ml Wasser, 0,38 ml 15%iger Natronlauge und 1,14 ml Wasser versetzt. Der Niederschlag wird abgesaugt, mit Tetrahydrofuran gewaschen und das Filtrat im Vakuum eingeengt. Aus einer Lösung in Aceton wird mit methanolischer Salzsäure das Dihydrochlorid gefällt.
Ausbeute: 0,80 g (62 % der Theorie),
Schmelzpunkt: 266-268 °C

| | | | | | | |
|---|---|---|---|---|---|---|
| Ber.: | C | 69,47 | H | 8,52 | N | 6,23 |
| Gef.: | | 69,42 | | 8,54 | | 5,98 |

Beispiel 3

4-(3-(3-(Carboxymethyl)piperidin-1-yl)prop-1-yl)-[2.2]paracyclophan-Natriumsalz

Zu 1,53 g (0,00364 Mol) 4-(3-(3-(Ethoxycarbonylmethyl)piperidin-1-yl)prop-1-yl)-[2.2]paracyclophan in 5 ml Ethanol werden 4 ml (0,004 Mol) 1 molare wässrige Natronlauge zugegeben. Danach wird 5 Stunden bei 80 °C gerührt, anschließend aus dem abgekühlten Reaktionsgemisch mit Aceton das Natriumsalz gefällt.
Ausbeute: 1,0 g (66 % der Theorie),
Schmelzpunkt: 228-235 °C

| | | | | | | |
|---|---|---|---|---|---|---|
| Ber.: | C | 75,52 | H | 7,80 | N | 3,39 |
| Gef.: | | 75,47 | | 7,80 | | 3,32 |

Beispiel 4

4-(2-Dimethylamino-eth-1-yl)-[2.2]paracyclophan-hydrochlorid

0,74 g (0,0024 Mol) 4-(2-Dimethylamino)-1,2-dioxo-eth-1-yl)-[2.2]paracyclophan werden in 10 ml absolutem Bortrifluoridetherat gegeben und alles auf 60 °C erwärmt. Bei dieser Temperatur werden nun 0,6 ml (0,006 Mol) Borandimethylsulfidkomplex (10 molare Lösung) zugetropft. Anschließend wird 5 Stunden unter Rückfluß erhitzt. Das abgekühlte Reaktionsgemisch wird tropfenweise mit Methanol versetzt, dann werden 10 ml methanolische Salzsäure zugegeben und nochmals 3 Stunden unter Rückfluß erhitzt. Der Ansatz wird nach dem Abdampfen des Lösungsmittels in Aceton gelöst und mit Ether das Hydrochlorid gefällt.
Ausbeute: 0,50 g (66 % der Theorie),
Schmelzpunkt: 194-196 °C

| | | | | | | |
|---|---|---|---|---|---|---|
| Ber.: | C | 76,05 | H | 8,30 | N | 4,43 |
| Gef.: | | 75,95 | | 8,32 | | 4,23 |

Beispiel 5

4-(3-(3-(2-Cyanoeth-1-yl)piperidin-1-yl)prop-1-yl)-[2.2]-paracyclophan-oxalat

Ein Gemisch von 0,34 Mol (0,00084 Mol) 4-(3-(3-(3-Amino-3-oxo-prop-1-yl)piperidin-1-yl)prop-1-yl)-[2.2]-paracyclophan und 0,92 g (0,006 Mol) Phosphoroxychlorid wird 40 Minuten auf 80 °C erhitzt. Das abgekühlt Reaktionsgemisch wird mit Wasser versetzt und mit konzentrierter Natronlauge alkalisch gestellt. Anschließend wird 3 mal mit Essigester extrahiert, über Magnesiumsulfat getrocknet und im Vakuum eingedampft. Aus einer Lösung in Essigester wird mit Oxalsäure das Oxalat gefällt.
Ausbeute: 0,24 g (60 % der Theorie),
Schmelzpunkt: 200-201 °C

| | | | | | | |
|---|---|---|---|---|---|---|
| Ber.: | C | 73,08 | H | 7,61 | N | 5,88 |
| Gef.: | | 72,96 | | 7,63 | | 5,90 |

Beispiel 6

4-(3-(Piperazin-1-yl)prop-1-yl)-[2.2]paracyclophan-dihydrochlorid

Ein Gemisch von 3,0 g (0,0074 Mol) 4-(3-(4-Ethoxycarbonyl-piperazin-1-yl)prop-1-yl)-[2.2]-paracyclophan, 20 ml Ethanol und 40 ml 8 molarer Natronlauge wird 12 Stunden unter Rückfluß erhitzt. Nach Einengen im Vakuum wird der erhaltene Rückstand 2 mal mit Essigester extrahiert, über Magnesiumsulfat getrocknet und im Vakuum eingedampft. Aus einer Lösung in Aceton wird mit methanolischer Salzsäure das Dihydrochlorid gefällt.
Ausbeute: 2,37 g (80 % der Theorie),
Schmelzpunkt: 274-278 °C

| | | | | | | |
|---|---|---|---|---|---|---|
| Ber.: | C | 67,80 | H | 7,92 | N | 6,88 |
| Gef.: | | 67,66 | | 8,06 | | 6,81 |

Beispiel 7

4-(3-(4-Acetyl-piperazin-1-yl)prop-1-yl)-[2.2]paracyclophan-hydrochlorid

0,67 g (0,002 Mol) 4-(3-(Piperazin-1-yl)-[2.2]paracyclophan und 0,30 ml (0,002 Mol) Triethylamin werden in 15 ml Methylenchlorid gelöst und unter Rühren 0,16 ml (0,0022 Mol) Acetylchlorid zugetropft. Nach 30 Minuten versetzt man mit Wasser. Die wässrige Phase wird 2 mal mit Methylenchlorid extrahiert. Die organische Phase wird über Magnesiumsulfat getrocknet und im Vakuum eingedampft. Der Rückstand wird in Aceton gelöst und daraus mit methanolischer Salzsäure das Hydrochlorid gefällt.
Ausbeute: 0,56 g (68 % der Theorie),
Schmelzpunkt: 153-158 ° C (Zers.)

| | | | | | | | |
|---|---|---|---|---|---|---|---|
| Ber.: | C | 72,70 | H | 8,05 | N | 6,78 |
| Gef.: | | 72,56 | | 8,31 | | 6,62 |

Beispiel 8

4-(3-Guanidinoprop-1-yl)-[2.2]paracyclophan-hydrochlorid

Zu einer Lösung von 2,3 g (0,012 Mol) 3,5-Dimethylpyrazol-1-carbonsäureamidinin 15 ml Dimethylformamid/Wasser (2/1) werden 0,7 g (0,002 Mol) 4-(3-Aminoprop-1-yl)-[2.2]paracyclophan-hydrochlorid und 0,5 g (0,005 Mol) Triethylamin gegeben. Die Lösung läßt man 5 Tage bei Raumtemperatur stehen und dampft anschließend das Lösungsmittel im Vakuum ab. Der Rückstand wird säulenchromatographisch über Kieselgel (Korngröße: 30-60 µm, Elutionsmittel: Methylenchlorid mit 10 % Ethanol) gereinigt.
Ausbeute: 0,21 g (27 % der Theorie),
Schmelzpunkt: 139 ° C

| | | | | | | | | |
|---|---|---|---|---|---|---|---|---|
| Ber.: | C | 61,50 | H | 7,16 | N | 12,05 | Cl | 14,32 |
| Gef.: | | 61,47 | | 7,17 | | 11,83 | | 13,96 |

Beispiel 9

4-(3-(N,N'-Dimethylamidino)prop-1-yl)-[2.2]paracyclophan-hydrochlorid

0,5 g (0,002 Mol) 4-(3-Cyanoprop-1-yl)-[2.2]paracyclophan werden in 10 ml absolutem Methanol und 20 ml absolutem Diethylether unter Stickstoff gelöst. Bei -5 ° C wird 30 Minuten Salzsäuregas eingeleitet und die Lösung eine Stunde bei Raumtemperatur nachgerührt. Anschließend wird das Lösungsmittel restlos abgedampft. Das erhaltene Öl wird in 20 ml absolutem Methanol gelöst und zu einer gesättigten Lösung von Methylamin in 35 ml absolutem Diethylether vorsichtig bei 0 ° C zugetropft. Das Gemisch wird 20 Stunden bei Raumtemperatur nachgerührt und dann im Vakuum eingedampft. Der Rückstand wird mit Diethylether verrieben und der entstandene Niederschlag abgesaugt und getrocknet.
Ausbeute: 0,41 g (64 % der Theorie),
Schmelzpunkt: 192 ° C

| | | | | | | |
|---|---|---|---|---|---|---|
| Ber.: | C | 73,25 | H | 8,23 | N | 7,62 |
| Gef.: | | 73,03 | | 8,19 | | 7,85 |

Beispiel 10

4-(2-(Piperidin-1-yl)ethoxy-[2.2]paracyclophan-oxalat

Hergestellt analog Beispiel 1 aus 4-(2-Chlorethoxy)-[2.2]-paracyclophan und Piperidin.
Ausbeute: 16 % der Theorie,

Schmelzpunkt: 186° C

| | | | | | | |
|---|---|---|---|---|---|---|
| Ber.: | C | 70,57 | H | 7,34 | N | 3,29 |
| Gef.: | | 70,43 | | 7,35 | | 3,43 |

Beispiel 11

4-(3-(Adamantyl-1-amino)prop-1-yl)-[2.2]paracyclcphan-hydrochlorid

Hergestellt analog Beispiel 1 aus 4-(3-Bromprop-1-yl)-[2.2]paracyclophan und 1-Adamantylamin.
Ausbeute: 31 % der Theorie,
Schmelzpunkt: 285° C

| | | | | | | | | |
|---|---|---|---|---|---|---|---|---|
| Ber.: | C | 79,87 | H | 8,78 | N | 3,21 | Cl | 8,13 |
| Gef.: | | 79,67 | | 8,99 | | 2,93 | | 8,33 |

Beispiel 12

4-(3-(3,5-Dimethyladamantyl-1-amino)prop-1-yl)-[2.2]paracyclophan-hydrochlorid

Hergestellt analog Beispiel 1 aus 4-(3-Bromprop-1-yl)-[2.2]paracyclophan und 3,5-Dimethyl-1-adamantylamin.
Ausbeute: 19 % der Theorie,
Schmelzpunkt: 301° C

| | | | | | | | | |
|---|---|---|---|---|---|---|---|---|
| Ber.: | C | 80,22 | H | 9,12 | N | 3,02 | Cl | 7,64 |
| Gef.: | | 79,94 | | 9,25 | | 2,86 | | 7,57 |

Beispiel 13

4-(3-(Piperidin-1-yl)propoxy)-[2.2]paracyclophan-hydrochlorid

Hergestellt analog Beispiel 1 aus 4-(3-Brompropoxy)-[2.2]paracyclophan und Piperidin.
Ausbeute: 46 % der Theorie,
Schmelzpunkt: 179° C

| | | | | | | | | |
|---|---|---|---|---|---|---|---|---|
| Ber.: | C | 74,68 | H | 8,36 | N | 3,63 | Cl | 9,18 |
| Gef.: | | 74,59 | | 8,32 | | 3,56 | | 9,37 |

Beispiel 14

4-(3-(3-(2-Dimethylamino-2-oxo-eth-1-yl)piperidin-1-yl)propoxy)-[2.2]paracyclophan-oxalat

Hergestellt analog Beispiel 1 aus 4-(3-Brompropoxy)-[2.2]paracyclophan und (Piperidin-3-yl)-essigsäuredimethylamid.
Ausbeute: 34 % der Theorie,

Schmelzpunkt: 169 °C

| | | | | | | |
|---|---|---|---|---|---|---|
| Ber.: | C | 68,68 | H | 7,69 | N | 5,34 |
| Gef.: | | 68,71 | | 7,77 | | 5,13 |

Beispiel 15

4-(3-(3-(2-Dimethylamino-2-oxo-eth-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und (Piperidin-3-yl)-essigsäuredimethylamid.
Ausbeute: 41 % der Theorie,
Schmelzpunkt: > 85 °C (Zers.)

| | | | | | | |
|---|---|---|---|---|---|---|
| Ber.: | C | 70,84 | H | 7,93 | N | 5,51 |
| Gef.: | | 71,02 | | 8,16 | | 5,38 |

Beispiel 16

4-(3-(3-(2-Ethoxycarbonyl-eth-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und 3-(Piperidin-3-yl)-propionsäureethylester.
Ausbeute: 70 % der Theorie,
Schmelzpunkt: 175 °C

| | | | | | | |
|---|---|---|---|---|---|---|
| Ber.: | C | 71,10 | H | 7,89 | N | 2,67 |
| Gef.: | | 71,11 | | 8,06 | | 2,72 |

Beispiel 17

4-(3-(3-(Hydroxycarbonyl)piperidin-1-yl)prop-1-yl)-[2.2]paracyclophan

Hergestellt analog Beispiel 3 aus 4-(3-(3-(Ethoxycarbonyl)piperidin-1-yl)prop-1-yl)-[2.2]paracyclophan und Natronlauge.
Ausbeute: 42 % der Theorie,
Schmelzpunkt: 128 °C

| | | | | | | |
|---|---|---|---|---|---|---|
| Ber.: | C | 79,54 | H | 8,28 | N | 3,71 |
| Gef.: | | 79,65 | | 8,38 | | 3,68 |

Beispiel 18

4-(3-(4-Acety-1-1,4-diazacyclohept-1-yl)prop-1-yl)-[2.2]paracyclophan-oxalat

Hergestellt analog Beispiel 7 aus 4-(3-(1,4-Diazacyclohept-1-yl)prop-1-yl)-[2.2]paracyclophan und Acetylchlorid.

Ausbeute: 40 % der Theorie,
Schmelzpunkt: 151 °C

| | | | | | | |
|---|---|---|---|---|---|---|
| Ber.: | C | 69,98 | H | 7,55 | N | 5,83 |
| Gef.: | | 69,81 | | 7,46 | | 5,66 |

Beispiel 19

4-(3-tert.Butylamino-prop-1-yl)-[2.2]paracyclophan-hydrochlorid

Hergestellt analog Beispiel 1 aus 4-(3-Bromprop-1-yl)-[2.2]paracyclophan und tert.Butylamin.
Ausbeute: 64 % der Theorie,
Schmelzpunkt: 251 °C

| | | | | | | | | |
|---|---|---|---|---|---|---|---|---|
| Ber.: | C | 77,17 | H | 9,01 | N | 3,91 | Cl | 9,90 |
| Gef.: | | 77,17 | | 9,14 | | 3,89 | | 9,69 |

Beispiel 20

4-(3-(3-(2-Hydroxyeth-1-yl)piperidin-1-yl)prop-1-yl)-[2.2]paracyclophan-hydrochlorid

Hergestellt analog Beispiel 2 aus 4-(3-(3-(Ethoxycarbonyl-methyl)piperidin-1-yl)prop-1-yl)-[2.2]-paracyclophan und Lithiumaluminiumhydrid.
Ausbeute: 75 % der Theorie,
Schmelzpunkt: 191 °C

| | | | | | | | | |
|---|---|---|---|---|---|---|---|---|
| Ber.: | C | 75,42 | H | 8,76 | N | 3,38 | Cl | 8,56 |
| Gef.: | | 75,32 | | 8,93 | | 3,20 | | 8,73 |

Beispiel 21

4-(3-(3-(N-Methansulfonyl-methylamino-methyl)piperidin-1-yl)prop-1-yl)-[2.2]paracyclophan-fumarat

Hergestellt analog Beispiel 7 aus 4-(3-(3-(Methylamino-methyl)piperidin-1-yl)prop-1-yl)-[2.2]-paracyclophan und Methansulfonsäurechlorid.
Ausbeute: 50 % der Theorie,
Schmelzpunkt: 110 °C (Zers.)

| | | | | | | | | |
|---|---|---|---|---|---|---|---|---|
| Ber.: | C | 65,24 | H | 7,42 | N | 4,91 | Cl | 5,62 |
| Gef.: | | 65,30 | | 7,62 | | 4,82 | | 5,47 |

Beispiel 22

4-(3-(N-(n-Octyl)methylamino)prop-1-yl)-[2.2]paracyclophan-oxalat

Hergestellt analog Beispiel 2 aus 4-(3-(N-(n-Octyl)methylamino)-3-oxo-prop-1-yl)-[2.2]paracyclophan und Lithiumaluminiumhydrid.

Ausbeute: 56 % der Theorie,
Schmelzpunkt: 141 °C

| | | | | | | |
|---|---|---|---|---|---|---|
| Ber.: | C | 74,81 | H | 9,00 | N | 2,91 |
| Gef.: | | 74,79 | | 8,89 | | 2,89 |

Beispiel 23

4-(3-(N-(2-Cyclohexyl-eth-1-yl)methylamino)prop-1-yl)-[2.2]paracyclophan-oxalat

Hergestellt analog Beispiel 1 aus 4-(3-Methylamino-prop-1-yl)-[2.2]paracyclophan und 1-Bromo-2-cyclohexylethan.
Ausbeute: 36 % der Theorie,
Schmelzpunkt: 134 °C

| | | | | | | |
|---|---|---|---|---|---|---|
| Ber.: | C | 75,12 | H | 8,62 | N | 2,92 |
| Gef.: | | 74,98 | | 8,59 | | 2,81 |

Beispiel 24

4-(3-(4-(Ethoxycarbonyl)piperazin-1-yl)prop-1-yl)-[2.2]paracyclophan-hydrochlorid

Hergestellt analog Beispiel 1 aus 4-(3-Bromprop-1-yl)-[2.2]paracyclophan und N-Ethoxycarbonyl-piperazin.
Ausbeute: 70 % der Theorie,
Schmelzpunkt: 229 °C (Zers.)

| | | | | | | | | |
|---|---|---|---|---|---|---|---|---|
| Ber.: | C | 70,49 | H | 7,96 | N | 6,32 | Cl | 8,00 |
| Gef.: | | 70,55 | | 8,06 | | 6,47 | | 8,18 |

Beispiel 25

4-(3-(3-(N-Acetyl-aminomethyl)piperidin-1-yl)prop-1-yl)-[2.2]paracyclophan-hydrochlorid

Hergestellt analog Beispiel 1 aus 4-(3-Bromprop-1-yl)-[2.2]paracyclophan und 3-(N-Acetyl-aminomethyl)piperidin.
Ausbeute: 37 % der Theorie,
Schmelzpunkt: 125 °C (Zers.)

| | | | | | | | | |
|---|---|---|---|---|---|---|---|---|
| Ber.: | C | 73,53 | H | 8,46 | N | 6,35 | Cl | 8,04 |
| Gef.: | | 73,40 | | 8,51 | | 6,28 | | 8,22 |

Beispiel 26

4-(3-(3-(N-Acetyl-methylamino-methyl)piperidin-1-yl)prop-1-yl)-[2.2]paracyclophan-hydrochlorid

Hergestellt analog Beispiel 1 aus 4-(3-Bromprop-1-yl)-[2.2]paracyclophan und 3-(N-Acetyl-methylamino-methyl)piperidin.

Ausbeute: 30 % der Theorie,
Schmelzpunkt: 105 °C

| | | | | | | | | |
|---|---|---|---|---|---|---|---|---|
| Ber.: | C | 73,90 | H | 8,64 | N | 6,16 | Cl | 7,79 |
| Gef.: | | 73,70 | | 8,73 | | 6,02 | | 7,85 |

Beispiel 27

4-(3-(3-(Hydroxymethyl)piperidin-1-yl)prop-1-yl)-[2.2]paracyclophan-hydrochlorid

Hergestellt analog Beispiel 1 aus 4-(3-Bromprop-1-yl)-[2.2]paracyclophan und 3-Hydroxymethylpiperidin.
Ausbeute: 50 % der Theorie,
Schmelzpunkt: 187 °C

| | | | | | | | | |
|---|---|---|---|---|---|---|---|---|
| Ber.: | C | 75,07 | H | 8,57 | N | 3,50 | Cl | 8,86 |
| Gef.: | | 74,93 | | 8,65 | | 3,44 | | 9,02 |

Beispiel 28

4-(3-(3-(Acetoxymethyl)piperidin-1-yl)prop-1-yl)-[2.2]paracyclophan-tartrat

Hergestellt analog Beispiel 7 aus 4-(3-(3-(Hydroxymethyl)piperidin-1-yl)prop-1-yl)-[2.2]paracyclophan und Acetylchlorid.
Ausbeute: 70 % der Theorie,
Schmelzpunkt: 65-70 °C (Zers.)

| | | | | | | |
|---|---|---|---|---|---|---|
| Ber.: | C | 67,01 | H | 7,44 | N | 2,52 |
| Gef.: | | 66,80 | | 7,56 | | 2,34 |

Beispiel 29

4-(3-(3-(Ethoxycarbonyl)piperidin-1-yl)prop-1-yl)-[2.2]paracyclophan-tartrat

Hergestellt analog Beispiel 1 aus 4-(3-Bromprop-1-yl)paracyclophan und (Piperidin-3-yl)-carbonsäureethylester.
Ausbeute: 35 % der Theorie,
Schmelzpunkt: 90-91 °C (Zers.)

| | | | | | | |
|---|---|---|---|---|---|---|
| Ber.: | C | 67,01 | H | 7,44 | N | 2,52 |
| Gef.: | | 66,81 | | 7,53 | | 2,49 |

Beispiel 30

4-(3-(4-(Ethoxycarbonyl)piperidin-1-yl)prop-1-yl)-[2.2]paracyclophan-tartrat

Hergestellt analog Beispiel 1 aus 4-(3-Bromprop-1-yl)-[2.2]paracyclophan und (Piperidin-4-yl)-

carbonsäureethylester.
Ausbeute: 30 % der Theorie,
Schmelzpunkt: >60° C (Zers.)

| | | | | | | |
|---|---|---|---|---|---|---|
| Ber.: | C | 67,01 | H | 7,44 | N | 2,52 |
| Gef.: | | 66,84 | | 7,32 | | 2,35 |

Beispiel 31

4-(3-[N-(2-(3,4-Dimethoxyphenyl)eth-1-yl)methylamino]prop-1-yl)-[2.2]paracyclophan-hydrochlorid

Hergestellt analog Beispiel 1 aus 4-(3-Bromprop-1-yl)-[2.2]paracyclophan und N-Methyl-homoveratrylamin.
Ausbeute: 30 % der Theorie,
Schmelzpunkt: 146° C

| | | | | | | | | |
|---|---|---|---|---|---|---|---|---|
| Ber.: | C | 75,05 | H | 7,98 | N | 2,92 | Cl | 7,39 |
| Gef.: | | 74,95 | | 8,10 | | 2,83 | | 7,14 |

Beispiel 32

4-(4-(2-(Diethylaminomethyl)piperidin-1-yl)but-1-yl)-[2.2]paracyclophan-dihydrochlorid

Hergestellt analog Beispiel 2 aus 4-(4-(2-(Diethylaminomethyl)piperidin-1-yl)-4-oxo-but-1-yl)-[2.2]-paracyclophan und Lithiumaluminiumhydrid.
Ausbeute: 41 % der Theorie,
Schmelzpunkt: >100° C (Zers.)

| | | | | | | | | |
|---|---|---|---|---|---|---|---|---|
| Ber.: | C | 71,26 | H | 9,17 | N | 5,54 | Cl | 14,03 |
| Gef.: | | 70,97 | | 9,28 | | 5,36 | | 13,96 |

Beispiel 33

4-(4-(4-Methylpiperazin-1-yl)but-1-yl)-[2.2]-paracyclophan-dihydrochlorid

Hergestellt analog Beispiel 2 aus 4-(4-(4-Methylpiperazin-1-yl)-4-oxo-but-1-yl)-[2.2]paracyclophan und Lithiumaluminiumhydrid.
Ausbeute: 66 % der Theorie,
Schmelzpunkt: 282° C

| | | | | | | | | |
|---|---|---|---|---|---|---|---|---|
| Ber.: | C | 68,95 | H | 8,33 | N | 6,43 | Cl | 16,28 |
| Gef.: | | 68,86 | | 8,25 | | 6,57 | | 16,35 |

Beispiel 34

4-(3-(Piperidin-1-yl)prop-1-yl)-[2.2]-paracyclophan-hydrochlorid

Hergestellt analog Beispiel 2 aus 4-(3-(Piperidin-1-yl)-3-oxo-prop-1-yl)-[2.2]paracyclophan und Lithiumaluminiumhydrid.
Ausbeute: 55 % der Theorie,
Schmelzpunkt: 244 ° C

| | | | | | | | | |
|---|---|---|---|---|---|---|---|---|
| Ber.: | C | 77,91 | H | 8,72 | N | 3,79 | Cl | 9,58 |
| Gef.: | | 77,80 | | 8,59 | | 3,86 | | 9,33 |

Beispiel 35

4-(3-(4-(2-(4-Trifluormethylphenoxy)eth-1-yl)piperazin-1-yl)prop-1-yl)-[2.2]paracyclophan-dihydrochlorid

Hergestellt analog Beispiel 1 aus 4-(3-(Piperazin-1-yl)prop-1-yl)-[2.2]paracyclophan und 1-Bromo-2-(4-trifluormethylphenoxy)ethan.
Ausbeute: 30 % der Theorie,
Schmelzpunkt: 241 ° C

| | | | | | | | | |
|---|---|---|---|---|---|---|---|---|
| Ber.: | C | 64,53 | H | 6,60 | N | 4,70 | Cl | 11,91 |
| Gef.: | | 64,64 | | 6,69 | | 4,54 | | 11,79 |

Beispiel 36

4-(3-(4-(2-(4-Fluorphenoxy)eth-1-yl)piperazin-1-yl)prop-1-yl)-[2.2]paracyclophan-dihydrochlorid

Hergestellt analog Beispiel 1 aus 4-(3-(Piperazin-1-yl)prop-1-yl)-[2.2]paracyclophan und 1-Bromo-2-(4-fluorphenoxy)ethan.
Ausbeute: 41 % der Theorie,
Schmelzpunkt: 254 ° C

| | | | | | | | | |
|---|---|---|---|---|---|---|---|---|
| Ber.: | C | 68,25 | H | 7,21 | N | 5,14 | Cl | 13,00 |
| Gef.: | | 68,40 | | 7,17 | | 5,20 | | 13,05 |

Beispiel 37

4-(3-(4-(2-(4-Nitrophenoxy)eth-1-yl)piperazin-1-yl)prop-1-yl)-[2.2]paracyclophan-dihydrochlorid

Hergestellt analog Beispiel 1 aus 4-(3-(Bromprop-1-yl)-[2.2]paracyclophanund 1-(2-(4-Nitrophenoxy)eth-1-yl)piperazin.
Ausbeute: 48 % der Theorie,
Schmelzpunkt: 241 ° C

| | | | | | | | | |
|---|---|---|---|---|---|---|---|---|
| Ber.: | C | 65,03 | H | 6,87 | N | 7,34 | Cl | 12,38 |
| Gef.: | | 65,00 | | 6,95 | | 7,24 | | 12,30 |

Beispiel 38

4-(5-(4-Methylpiperazin-1-yl)pent-1-yl)-[2.2]paracyclophan-dihydrochlorid

Hergestellt analog Beispiel 2 aus 4-(5-(4-Methylpiperazin-1-yl)-5-oxo-pent-1-yl)-[2.2]paracyclophan und Lithiumaluminiumhydrid.
Ausbeute: 62 % der Theorie,
Schmelzpunkt: 267° C

| | | | | | | | | |
|---|---|---|---|---|---|---|---|---|
| Ber.: | C | 69,47 | H | 8,52 | N | 6,23 | Cl | 15,78 |
| Gef.: | | 69,42 | | 8,54 | | 6,10 | | 16,00 |

Beispiel 39

4-(3-(4-Methylpiperazin-1-yl)prop-1-en-1-yl)-[2.2]paracyclophan-dihydrochlorid

Hergestellt analog Beispiel 2 aus 4-(3-(4-Methylpiperazin-1-yl)-3-oxo-prop-1-en-1-yl)-[2.2]paracyclophan und Lithiumaluminiumhydrid.
Ausbeute: 63 % der Theorie,
Schmelzpunkt: 239° C

| | | | | | | | | |
|---|---|---|---|---|---|---|---|---|
| Ber.: | C | 68,72 | H | 7,69 | N | 6,68 | Cl | 16,91 |
| Gef.: | | 68,59 | | 7,80 | | 6,45 | | 16,86 |

Beispiel 40

4-(3-(2-(Diethylaminomethyl)piperidin-1-yl)prop-1-yl)-[2.2]paracyclophan-dihydrochlorid

Hergestellt analog Beispiel 2 aus 4-(3-(2-(Diethylaminomethyl)piperidin-1-yl)-3-oxo-prop-1-yl)-[2.2]-paracyclophan und Lithiumaluminiumhydrid.
Ausbeute: 43 % der Theorie,
Schmelzpunkt: 149-151° C

| | | | | | | | | |
|---|---|---|---|---|---|---|---|---|
| Ber.: | C | 70,85 | H | 9,02 | N | 5,70 | Cl | 14,43 |
| Gef.: | | 70,37 | | 8,91 | | 5,65 | | 14,30 |

Beispiel 41

4-(3-(4-(3-Phenylprop-2-en-1-yl)piperazin-1-yl)prop-1-yl)-[2.2]paracyclophan-dihydrochlorid

Hergestellt analog Beispiel 2 aus 4-(3-(4-(3-Phenylprop-2-en-1-yl)piperazin-1-yl)-3-oxo-prop-1-yl)-[2.2]-paracyclophan und Lithiumaluminiumhydrid.
Ausbeute: 49 % der Theorie,
Schmelzpunkt: 253-255° C

| | | | | | | | | |
|---|---|---|---|---|---|---|---|---|
| Ber.: | C | 73,41 | H | 7,70 | N | 5,35 | Cl | 13,54 |
| Gef.: | | 73,22 | | 7,79 | | 5,67 | | 13,32 |

Beispiel 42

4-(3-(4-Methylpiperazin-1-yl)prop-1-yl)-[2.2]paracyclophan-dihydrochlorid

Hergestellt analog Beispiel 2 aus 4-(3-(4-Methylpiperazin-1-yl)-3-oxo-prop-1-yl)-[2.2]paracyclophan und Lithiumaluminiumhydrid.
Ausbeute: 76 % der Theorie,
Schmelzpunkt: 262-263 °C

| | | | | | | | | |
|---|---|---|---|---|---|---|---|---|
| Ber.: | C | 68,39 | H | 8,13 | N | 6,65 | Cl | 16,83 |
| Gef.: | | 68,27 | | 8,23 | | 6,61 | | 16,61 |

Beispiel 43

4-(3-Dimethylamino-prop-1-yl)-[2.2]paracyclophan-dihydrochlorid

Hergestellt analog Beispiel 2 aus 4-(3-Dimethylamino-3-oxo-prop-1-yl)-[2.2]paracyclophan und Lithiumaluminiumhydrid.
Ausbeute: 72 % der Theorie,
Schmelzpunkt: 99-101 °C

| | | | | | | | | |
|---|---|---|---|---|---|---|---|---|
| Ber.: | C | 76,45 | H | 8,55 | N | 4,25 | Cl | 10,75 |
| Gef.: | | 76,35 | | 8,67 | | 4,19 | | 10,76 |

Beispiel 44

4-(3-Methylamino-prop-1-yl)-[2.2]paracyclophan-hydrochlorid

Hergestellt analog Beispiel 2 aus 4-(3-Methylamino-3-oxo-prop-1-yl)-[2.2]paracyclophan und Lithiumaluminiumhydrid.
Ausbeute: 62 % der Theorie,
Schmelzpunkt: 215-218 °C

| | | | | | | | | |
|---|---|---|---|---|---|---|---|---|
| Ber.: | C | 76,05 | H | 8,30 | N | 4,43 | Cl | 11,22 |
| Gef.: | | 75,95 | | 8,55 | | 4,17 | | 11,19 |

Beispiel 45

4-(2-(4-Benzylpiperazin-1-yl)eth-1-yl)-[2.2]paracyclophan-dihydrochlorid

Hergestellt analog Beispiel 2 aus 4-(2-(4-Benzylpiperazin-1-yl)-2-oxo-eth-1-yl)-[2.2]paracyclophan und Lithiumaluminiumhydrid.
Ausbeute: 80 % der Theorie,
Schmelzpunkt: 245-248 °C

| | | | | | | | | |
|---|---|---|---|---|---|---|---|---|
| Ber.: | C | 72,04 | H | 7,50 | N | 5,79 | Cl | 14,67 |
| Gef.: | | 72,14 | | 7,59 | | 5,88 | | 14,60 |

Beispiel 46

4-(2-(4-(3-Phenyl-prop-2-en-1-yl)piperazin-1-yl)eth-1-yl)-[2.2]paracyclophan-dihydrochlorid

Hergestellt analog Beispiel 2 aus 4-(2-(4-(3-Phenyl-1-oxo-prop-2-en-1-yl)piperazin-1-yl)eth-1-yl)-[2.2]-paracyclophan und Lithiumaluminiumhydrid.
Ausbeute: 40 % der Theorie,
Schmelzpunkt: 252-254 °C

| | | | | | | | | |
|---|---|---|---|---|---|---|---|---|
| Ber.: | C | 73,07 | H | 7,52 | N | 5,50 | Cl | 13,91 |
| Gef.: | | 72,90 | | 7,62 | | 5,34 | | 13,71 |

Beispiel 47

4-(2-(4-Methylpiperazin-1-yl)eth-1-yl)-[2.2]paracyclophan-dihydrochlorid

Hergestellt analog Beispiel 2 aus 4-(2-(4-Methylpiperazin-1-yl)-2-oxo-eth-1-yl)-[2.2]paracyclophan und Lithiumaluminiumhydrid.
Ausbeute: 69 % der Theorie,
Schmelzpunkt: 268-270 °C

| | | | | | | | | |
|---|---|---|---|---|---|---|---|---|
| Ber.: | C | 67,80 | H | 7,92 | N | 6,88 | Cl | 17,40 |
| Gef.: | | 67,93 | | 7,81 | | 6,94 | | 17,65 |

Beispiel 48

4-(2-(N-Morpholino)eth-1-yl)-[2.2]paracyclophan-dihydrochlorid

Hergestellt analog Beispiel 2 aus 4-(2-(N-Morpholino)-2-oxo-eth-1-yl)-[2.2]paracyclophan und Lithiumaluminiumhydrid.
Ausbeute: 63 % der Theorie,
Schmelzpunkt: 105-107 °C

| | | | | | | |
|---|---|---|---|---|---|---|
| Ber.: | C | 82,20 | H | 8,47 | N | 4,36 |
| Gef.: | | 82,18 | | 8,50 | | 4,26 |

Beispiel 49

4-(2-(Piperidin-1-yl)eth-1-yl)-[2.2]paracyclophan-hydrochlorid

Hergestellt analog Beispiel 2 aus 4-(2-(Piperidin-1-yl)-2-oxo-eth-1-yl)-[2.2]paracyclophan und Lithiumaluminiumhydrid.
Ausbeute: 60 % der Theorie,
Schmelzpunkt: 249-252 °C

| | | | | | | | | |
|---|---|---|---|---|---|---|---|---|
| Ber.: | C | 77,61 | H | 8,50 | N | 3,94 | Cl | 9,96 |
| Gef.: | | 77,60 | | 8,58 | | 3,76 | | 10,10 |

Beispiel 50

4-(2-(Pyrrolidin-1-yl)eth-1-yl)-[2.2]paracyclophan-hydrochlorid

Hergestellt analog Beispiel 2 aus 4-(2-(Pyrrolidin-1-yl)-2-oxo-eth-1-yl)-[2.2]paracyclophan und Lithiumaluminiumhydrid.
Ausbeute: 63 % der Theorie,
Schmelzpunkt: 231-233 °C

| | | | | | | | | |
|---|---|---|---|---|---|---|---|---|
| Ber.: | C | 77,28 | H | 8,25 | N | 4,10 | Cl | 10,37 |
| Gef.: | | 77,16 | | 8,32 | | 3,99 | | 10,23 |

Beispiel 51

4-(2-(Di-n-propylamino)eth-1-yl)-[2.2]paracyclophan-hydrochlorid

Hergestellt analog Beispiel 2 aus 4-(2-(Di-n-propylamino)-2-oxo-eth-1-yl)-[2.2]paracyclophan und Lithiumaluminiumhydrid.
Ausbeute: 54 % der Theorie,
Schmelzpunkt: 168-169 °C

| | | | | | | | | |
|---|---|---|---|---|---|---|---|---|
| Ber.: | C | 77,49 | H | 9,21 | N | 3,77 | Cl | 9,53 |
| Gef.: | | 77,31 | | 9,28 | | 3,63 | | 9,71 |

Beispiel 52

4-Dimethylaminomethyl-[2.2]paracyclophan-hydrochlorid

Hergestellt analog Beispiel 2 aus 4-Dimethylaminocarbonyl-[2.2]paracyclophan und Lithiumaluminiumhydrid.
Ausbeute: 60 % der Theorie,
Schmelzpunkt: 242-243 °C

| | | | | | | | | |
|---|---|---|---|---|---|---|---|---|
| Ber.: | C | 75,60 | H | 8,01 | N | 4,64 | Cl | 11,75 |
| Gef.: | | 75,49 | | 7,99 | | 4,45 | | 11,50 |

Beispiel 53

4-Methylaminomethyl-[2.2]paracyclophan-hydrochlorid

Hergestellt analog Beispiel 2 aus 4-Methylaminocarbonyl-[2.2]paracyclophan und Lithiumaluminiumhydrid.
Ausbeute: 70 % der Theorie,
Schmelzpunkt: 249-250 °C

| | | | | | | | | |
|---|---|---|---|---|---|---|---|---|
| Ber.: | C | 75,11 | H | 7,70 | N | 4,87 | Cl | 12,32 |
| Gef.: | | 74,97 | | 7,56 | | 4,72 | | 12,04 |

Beispiel 54

4-(2-Diethylamino-eth-1-yl)-[2.2]paracyclophan-hydrochlorid

Hergestellt analog Beispiel 2 aus 4-(2-Diethylamino-2-oxo-eth-1-yl)-[2.2]paracyclophan und Lithiumaluminiumhydrid.
Ausbeute: 37 % der Theorie,
Schmelzpunkt: 215-217 °C

| | | | | | | | | |
|---|---|---|---|---|---|---|---|---|
| Ber.: | C | 76,83 | H | 8,79 | N | 4,07 | Cl | 10,31 |
| Gef.: | | 76,72 | | 8,75 | | 4,06 | | 10,26 |

Beispiel 55

4-(2-Methylamino-eth-1-yl)-[2.2]paracyclophan-hydrochlorid

Hergestellt analog Beispiel 4 aus 4-(2-(Methylamino)-1,2-di-oxo-eth-1-yl)-[2.2]paracyclophan und Borandimethylsulfidkomplex (10 molare Lösung).
Ausbeute: 63 % der Theorie,
Schmelzpunkt: 280-281 °C

| | | | | | | | | |
|---|---|---|---|---|---|---|---|---|
| Ber.: | C | 75,60 | H | 8,01 | N | 4,64 | Cl | 11,75 |
| Gef.: | | 75,40 | | 7,95 | | 4,42 | | 11,57 |

Beispiel 56

4-(2-Aminoeth-1-yl)-[2.2]paracyclophan-hydrochlorid

Hergestellt analog Beispiel 4 aus 4-(2-Amino-1,2-dioxo-eth-1-yl)-[2.2]paracyclophan und Borandimethylsulfidkomplex (10 molarer Lösung).
Ausbeute: 40 % der Theorie,
Schmelzpunkt: 150-152 °C

| | | | | | | | | |
|---|---|---|---|---|---|---|---|---|
| Ber.: | C | 75,11 | H | 7,70 | N | 4,87 | Cl | 12,32 |
| Gef.: | | 75,06 | | 8,06 | | 4,84 | | 12,12 |

Beispiel 57

4-(2-tert.Butylamino-eth-1-yl)-[2.2]paracyclophan-hydrochlorid

Hergestellt analog Beispiel 4 aus 4-(2-tert.Butylamino-1,2-dioxo-eth-1-yl)-[2.2]paracyclophan und Borandimethylsulfidkomplex (10 molare Lösung).
Ausbeute: 40 % der Theorie,
Schmelzpunkt: 276-279 °C

| | | | | | | | | |
|---|---|---|---|---|---|---|---|---|
| Ber.: | C | 76,83 | H | 8,79 | N | 4,07 | Cl | 10,31 |
| Gef.: | | 76,66 | | 8,95 | | 3,93 | | 10,18 |

Beispiel 58

4-(3-(4-(2-(3,4,5-Trimethoxyphenyl)eth-1-yl)piperazin-1-yl)prop-1-yl)-[2.2]paracyclophan-dihydrochlorid

Hergestellt analog Beispiel 1 aus 4-(3-Bromprop-1-yl)-[2.2]paracyclophan und 1-(2-(3,4,5-Trimethoxyphenyl)eth-1-yl)piperazin.
Ausbeute: 51 % der Theorie,
Schmelzpunkt: 260-262 °C

| | | | | | | | | |
|---|---|---|---|---|---|---|---|---|
| Ber.: | C | 67,87 | H | 7,71 | N | 4,66 | Cl | 11,79 |
| Gef.: | | 67,66 | | 7,74 | | 4,51 | | 11,81 |

Beispiel 59

4-(3-(4-(2-(p-Tolyloxy)eth-1-yl)piperazin-1-yl)prop-1-yl)-[2.2]paracyclophan-dihydrochlorid

Hergestellt analog Beispiel 1 aus 4-(3-Bromprop-1-yl)-[2.2]paracyclophan und 1-(2-(p-Tolyloxy)eth-1-yl)-piperazin.
Ausbeute: 52 % der Theorie,
Schmelzpunkt: 258-260 °C

| | | | | | | | | |
|---|---|---|---|---|---|---|---|---|
| Ber.: | C | 70,96 | H | 7,82 | N | 5,17 | Cl | 13,09 |
| Gef.: | | 70,83 | | 8,05 | | 5,09 | | 12,85 |

Beispiel 60

4-(3-(3-Piperidin-1-yl)prop-1-in-1-yl)-[2.2]paracyclophan-dioxalat

Hergestellt analog Beispiel 1 aus 4-(3-Chlorprop-1-in-yl)-[2.2]paracyclophan und Piperidin.
Ausbeute: 38 % der Theorie,
Schmelzpunkt: 150-151 °C

| | | | | | | |
|---|---|---|---|---|---|---|
| Ber.: | C | 66,00 | H | 6,13 | N | 2,75 |
| Gef.: | | 67,89 | | 6,27 | | 3,09 |

Beispiel 61

4-(3-(4-(3-Dimethylamino-3-oxo-prop-1-yl)piperidin-1-yl)-prop-1-yl)-[2.2]paracyclophan-oxalat

Hergestellt analog Beispiel 1 aus 4-(3-Bromprop-1-yl)-[2.2]paracyclophan und 3-(Piperidin-4-yl)-propionsäuredimethylamid.
Ausbeute: 20 % der Theorie,
Schmelzpunkt: 175 °C (Zers.)

| | | | | | | |
|---|---|---|---|---|---|---|
| Ber.: | C | 71,24 | H | 8,10 | N | 5,36 |
| Gef.: | | 71,15 | | 8,24 | | 5,31 |

Beispiel 62

4-(3-(4-(3-Dimethylamino-4-oxo-but-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und 3-(Piperidin-4-yl)-buttersäuredimethylamid.
Ausbeute: 46 % der Theorie,
Schmelzpunkt: 185° C

| | | | | | | |
|---|---|---|---|---|---|---|
| Ber.: | C | 71,61 | H | 8,26 | N | 5,22 |
| Gef.: | | 71,40 | | 8,20 | | 5,14 |

Beispiel 63

4-(3-(4-(2-Dimethylamino-2-oxo-eth-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und (Piperidin-4-yl)-essigsäuredimethylamid.
Ausbeute: 38 % der Theorie,
Schmelzpunkt: 229° C (Zers.)

| | | | | | | |
|---|---|---|---|---|---|---|
| Ber.: | C | 70,84 | H | 7,93 | N | 5,51 |
| Gef.: | | 70,75 | | 7,83 | | 5,64 |

Beispiel 64

4-(3-(3-(5-Dimethylamino-5-oxo-pent-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und 5-(Piperidin-3-yl)-pentansäuredimethylamid.
Ausbeute: 54 % der Theorie,
Schmelzpunkt: 160° C

| | | | | | | |
|---|---|---|---|---|---|---|
| Ber.: | C | 71,97 | H | 8,42 | N | 5,09 |
| Gef.: | | 71,79 | | 8,37 | | 5,04 |

Beispiel 65

4-(3-(3-(3-Amino-3-oxo-prop-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und 3-(Piperidin-3-yl)-propionsäureamid.
Ausbeute: 50 % der Theorie,
Schmelzpunkt: 186° C

| | | | | | | |
|---|---|---|---|---|---|---|
| Ber.: | C | 70,42 | H | 7,74 | N | 5,66 |
| Gef.: | | 70,52 | | 8,07 | | 5,71 |

Beispiel 66

4-(3-(4-Carboxy-piperidin-1-yl)prop-1-yl)-[2.2]paracyclophan-Natriumsalz

Hergestellt analog Beispiel 3 aus 4-(3-(4-Ethoxycarbonyl-piperidin-1-yl)prop-1-yl)-[2.2]paracyclophan und Natronlauge.
Ausbeute: 68 % der Theorie,
Schmelzpunkt: ~ 280 ° C

| | | | | | | |
|---|---|---|---|---|---|---|
| Ber.: | C | 75,16 | H | 7,57 | N | 3,51 |
| Gef.: | | 74,97 | | 7,77 | | 3,77 |

Beispiel 67

4-(3-(4-(Dimethylaminocarbonyl)piperidin-1-yl)prop-1-yl)-[2.2]paracyclophan-oxalat

Hergestellt analog Beispiel 1 aus 4-(3-Brom-prop-1-yl)-[2.2]paracyclophan und (Piperidin-4-yl)-carbonsäuredimethylamid.
Ausbeute: 36 % der Theorie,
Schmelzpunkt: 170 ° C (Zers.)

| | | | | | | |
|---|---|---|---|---|---|---|
| Ber.: | C | 70,42 | H | 7,74 | N | 5,66 |
| Gef.: | | 70,27 | | 7,79 | | 5,69 |

Beispiel 68

4-(3-(3-(Dimethylaminocarbonyl)piperidin-1-yl)prop-1-yl)-[2.2]paracyclophan-oxalat

Hergestellt analog Beispiel 1 aus 4-(3-Bromprop-1-yl)-[2.2]paracyclophan und (Piperidin-3-yl)-carbonsäuredimethylamid.
Ausbeute: 23 % der Theorie,
Schmelzpunkt: 140 ° C

| | | | | | | |
|---|---|---|---|---|---|---|
| Ber.: | C | 70,42 | H | 7,74 | N | 5,66 |
| Gef.: | | 70,26 | | 7,72 | | 5,73 |

Beispiel 69

4-(3-(3-(2-Carboxy-eth-1-yl)piperidin-1-yl)prop-1-yl)-[2.2]paracyclophan-Natriumsalz

Hergestellt analog Beispiel 3 aus 4-(3-(3-(2-Ethoxycarbonyl-eth-1-yl)piperidin-1-yl)prop-1-yl)-[2.2]-paracyclophan und Natronlauge.
Ausbeute: 80 % der Theorie,
Schmelzpunkt: 262 ° C

| | | | | | | |
|---|---|---|---|---|---|---|
| Ber.: | C | 75,84 | H | 8,02 | N | 3,28 |
| Gef.: | | 75,66 | | 8,13 | | 3,18 |

Beispiel 70

4-(3-(3-(3-Dimethylamino-3-oxo-prop-1-yl)piperidin-1-yl)prop-1-yl)-[2.2]paracyclophan-oxalat

Hergestellt analog Beispiel 1 aus 4-(3-Bromprop-1-yl)-[2.2]paracyclophan und 3-(Piperidin-3-yl)-propionsäuredimethylamid.
Ausbeute: 43 % der Theorie,
Schmelzpunkt: 146 °C

| | | | | | | |
|---|---|---|---|---|---|---|
| Ber.: | C | 71,24 | H | 8,10 | N | 5,36 |
| Gef.: | | 71,07 | | 8,08 | | 5,56 |

Beispiel 71

4-Methansulfonyloxy-7-(3-(piperidin-1-yl)prop-1-yl)-[2.2]paracyclophan-oxalat

Hergestellt analog Beispiel 7 aus 4-Hydroxy-7-(3-(piperidin-1-yl)prop-1-yl)-[2.2]paracyclophan und Methansulfonsäurechlorid.
Ausbeute: 30 % der Theorie,
Schmelzpunkt: 191 °C

| | | | | | | | | |
|---|---|---|---|---|---|---|---|---|
| Ber.: | C | 62,65 | H | 6,82 | N | 2,71 | S | 6,19 |
| Gef.: | | 62,52 | | 6,83 | | 2,69 | | 6,43 |

Beispiel 72

4-Methoxy-7-(3-(piperidin-1-yl)prop-1-yl)-[2.2]paracyclophan-hydrobromid

Hergestellt analog Beispiel 1 aus 4-Methoxy-7-(3-bromprop-1-yl)-[2.2]paracyclophan und Piperidin.
Ausbeute: 63 % der Theorie,
Schmelzpunkt: 196-197 °C

| | | | | | | | | |
|---|---|---|---|---|---|---|---|---|
| Ber.: | C | 67,56 | H | 7,71 | N | 3,15 | Br | 17,98 |
| Gef.: | | 67,44 | | 7,65 | | 3,32 | | 18,19 |

Beispiel 73

4-Methoxy-7-(3-(3-(N-acetyl-methylamino-methyl)piperidin-1-yl)prop-1-yl)-[2.2]paracyclophan-oxalat

Hergestellt analog Beispiel 1 aus 4-Methoxy-7-(3-bromprop-1-yl)-[2.2]paracyclophan und 3-(N-Acetyl-methylamino-methyl)piperidin.
Ausbeute: 60 % der Theorie,
Schmelzpunkt: 140-143 °C

| | | | | | | |
|---|---|---|---|---|---|---|
| Ber.: | C | 69,12 | H | 7,86 | N | 5,20 |
| Gef.: | | 69,31 | | 7,71 | | 5,10 |

Beispiel 74

4-Hydroxy-7-(3-(3-(N-acetyl-methylamino-methyl)piperidin-1-yl)prop-1-yl)[2.2]paracyclophan-oxalat

Hergestellt analog Beispiel 1 aus 4-Hydroxy-7-(3-bromprop-1-yl)-[2.2]paracyclophan und 3-(N-Acetyl-methylamino-methyl)piperidin.
Ausbeute: 60 % der Theorie,
Schmelzpunkt: 60° C (Zers.)

| | | | | | | |
|---|---|---|---|---|---|---|
| Ber.: | C | 68,76 | H | 7,69 | N | 5,34 |
| Gef.: | | 68,97 | | 7,87 | | 5,16 |

Beispiel 75

4-Hydroxy-7-(3-(piperidin-1-yl)prop-1-yl)-[2.2]paracyclophan

Hergestellt analog Beispiel 1 aus 4-Hydroxy-7-(3-bromprop-1-yl)-[2.2]paracyclophan und Piperidin.
Ausbeute: 63 % der Theorie,
Schmelzpunkt: 143-145° C

| | | | | | | |
|---|---|---|---|---|---|---|
| Ber.: | C | 82,47 | H | 8,94 | N | 4,01 |
| Gef.: | | 82,23 | | 8,99 | | 4,10 |

Beispiel 76

4-Methyl-7-(3-(3-(N-acetyl-methylamino-methyl)piperidin-1-yl)prop-1-yl)-[2.2]paracyclophan-oxalat

Hergestellt analog Beispiel 1 aus 4-Methyl-7-(3-bromprop-1-yl)-[2.2]paracyclophan und 3-(N-Acetyl-methylamino-methyl)piperidin.
Ausbeute: 49 % der Theorie,
Schmelzpunkt: 153° C

| | | | | | | |
|---|---|---|---|---|---|---|
| Ber.: | C | 71,24 | H | 8,04 | N | 5,36 |
| Gef.: | | 71,07 | | 8,23 | | 5,27 |

Beispiel 77

4,5,12,13-Tetramethyl-7-(3-(3-(N-acetyl-methylamino-methyl)piperidin-1-yl)prop-1-yl)-[2.2]paracyclophan-oxalat

Hergestellt analog Beispiel 1 aus 4,5,12,13-Tetramethyl-7-(3-bromprop-1-yl)-[2.2]paracyclophan und 3-(N-Acetyl-methylamino-methyl)piperidin.
Ausbeute: 60 % der Theorie,
Schmelzpunkt: ~ 160° C

| | | | | | | |
|---|---|---|---|---|---|---|
| Ber.: | C | 72,31 | H | 8,57 | N | 4,96 |
| Gef.: | | 72,17 | | 8,74 | | 5,02 |

Beispiel 78

4,5,12,13-Tetramethyl-7-(3-(3-(N-acetyl-methylamino-methyl)piperidin-1-yl)prop-1-yl)-[2.2]paracyclophan-oxalat

Hergestellt analog Beispiel 1 aus 4,5,12,13-Tetramethyl-7-(3-bromprop-1-yl)-[2.2]paracyclophan und 3-(N-Acetyl-methylamino-methyl)piperidin.
Ausbeute: 44 % der Theorie,
Schmelzpunkt: 178 °C (Zers.)

| | | | | | | |
|---|---|---|---|---|---|---|
| Ber.: | C | 72,31 | H | 8,57 | N | 4,96 |
| Gef.: | | 72,13 | | 8,74 | | 4,87 |

Beispiel 79

(S)(+) 4-(3-(Piperidin-1-yl)prop-1-yl)-[2.2]paracyclophan-hydrochlorid

Zu 1,50 g (0,01 Mol) L(+) Weinsäure in 60 ml Ethanol werden 3,3 g (0,01 Mol) 4-(3-(Piperidin-1-yl)-prop-1-yl)-[2.2]paracyclophan, gelöst in 10 ml Ethanol, gegeben. Danach wird 5 Minuten unter Rückfluß erhitzt und abgekühlt. Der entstandene Niederschlag wird abfiltriert und 4 x aus 95%igem wässrigem Ethanol umkristallisiert. Anschließend wird das Salz mit 2 molarer Natronlauge freigesetzt und die freie Base dreimal mit Essigester extrahiert. Die organische Phase wird über Natriumsulfat getrocknet und eingedampft. Das Hydrochlorid wird aus Aceton mit methanolischer Salzsäure gefällt.
Ausbeute: 0,70 g (42 % der Theorie),
Schmelzpunkt: 262 °C
$[\alpha]_D^{20}$ = + 64° (c = 0,70 in Chloroform).

**Patentansprüche**

1. Cyclophane der allgemeinen Formel

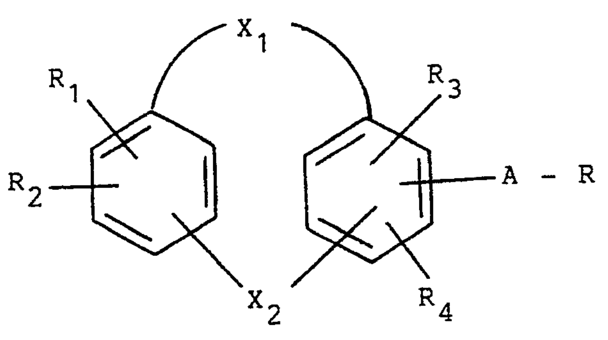

,(I)

in der
$X_1$ und $X_2$, die gleich oder verschieden sein können, eine geradkettige Alkylen- oder Alkenylengruppe mit jeweils 2 bis 4 Kohlenstoffatomen,

A eine geradkettige Alkylengruppe mit 1 bis 6 Kohlenstoffatomen, eine $-Y_1-A_1-$ oder $-Y_2-A_2-$Gruppe, in denen
$Y_1$ ein Sauerstoffatom, eine Sulfenyl-, Sulfinyl- oder Sulfonylgruppe und
$A_1$ eine geradkettige Alkylengruppe mit 2 bis 4 Kohlenstoffatomen,
$Y_2$ eine Ethenylen- oder Ethinylengruppe und
$A_2$ eine geradkettige Alkylengruppe mit 1 bis 3 Kohlenstoffatomen bedeuten,

wobei die Reste $A_1$ und $A_2$ mit dem Rest R verknüpft sind und das Kohlenstoffgerüst der Gruppen A, $A_1$ und $A_2$ zusätzlichdurch eine oder zwei Methylgruppen substituiert sein kann,

$R_1$, $R_2$, $R_3$ und $R_4$, die gleich oder verschieden sein können, ein Wasserstoff- oder Halogenatom, eine Alkyl-, Hydroxy-, Alkoxy- oder Alkylsulfonyloxygruppe mit jeweils 1 bis 3 Kohlenstoffatomen im Alkylteil und

R eine Cyanogruppe oder eine Gruppe der Formeln

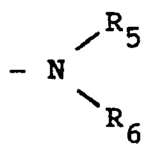

oder

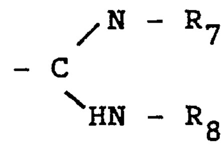

bedeuten, in denen
$R_5$ ein Wasserstoffatom, eine Alkylgruppe mit 1 bis 10 Kohlenstoffatomen, eine Cycloalkylgruppe mit 5 bis 7 Kohlenstoffatomen, eine bi- oder tricyclische Alkylgruppe mit 6 bis 15 Kohlenstoffatomen, wobei jeder der vorstehend erwähnten cyclischen Reste durch eine oder zwei Alkylgruppen mit jeweils 1 bis 3 Kohlenstoffatomen substituiert sein kann, oder eine Phenylalkylgruppe, in der der Alkylteil 1 bis 3 Kohlenstoffatome enthalten kann und der Phenylkern durch ein Halogenatom, durch eine Alkyl- oder Alkoxygruppe mit jeweils 1 bis 3 Kohlenstoffatomen mono-, di- oder trisubstituiert sein kann,

$R_6$ ein Wasserstoffatom oder eine Alkylgruppe mit 1 bis 5 Kohlenstoffatomen oder

$R_5$ und $R_6$ zusammen mit dem dazwischenliegenden Stickstoffatom eine Pyrrolidino-, Piperidino-, Hexamethylenimino- oder Morpholinogruppe, wobei

i) in einer vorstehend bei der Definition der Reste $R_5$ und $R_6$ erwähnten Piperidino- oder Hexamethyleniminogruppe eine Methylengruppe in 4-Stellung durch eine Imino-, Alkylimino-, Alkoxycarbonylimino-, Alkanoylimino- oder Phenylalkyliminogruppe oder durch eine in 2- oder 3-Stellung durch eine Phenoxygruppe substituierte Alkyliminogruppe mit 2 oder 3 Kohlenstoffatomen oder durch eine Phenylalkenyliminogruppe, in welcher der Alkenylteil 2 oder 3 Kohlenstoffatome enthalten kann, ersetzt sein kann oder
ii) eine vorstehend bei der Definition der Reste $R_5$ und $R_6$ erwähnte Piperidinogruppe durch eine Hydroxy-, Alkoxy-, Carboxy-, Alkoxycarbonyl-, Aminocarbonyl-, Alkylaminocarbonyl-oder Dialkylaminocarbonylgruppe oder durch eine Alkylgruppe mit 1 bis 5 Kohlenstoffatomen, welche durch eine Hydroxy-, Alkoxy-, Alkanoyloxy-, Cyano-, Carboxy-, Alkoxycarbonyl-, Aminocarbonyl-, Alkylaminocarbonyl-, Dialkylaminocarbonyl-, Amino-, Alkylamino-, Dialkylamino-, Alkanoylamino-, Alkylsulfonylamino-, N-Alkanoyl-alkylamino- oder N-Alkylsulfonyl-alkylaminogruppe substituiert sein kann, wobei, soweit nichts anderes erwähnt wurde, die unter i) und ii) vorstehend erwähnten Alkyl-, Alkoxy- und Alkanoylteile jeweils 1 bis 3 Kohlenstoffatome enthalten können und die vorstehend erwähnten Phenylkerne jeweils durch eine Nitro- oder Trifluormethylgruppe monosubstituiert oder durch Halogenatome, Alkylgruppen mit 1 bis 3 Kohlenstoffatomen oder Alkyloxygruppen mit 1 bis 3 Kohlenstoffatomen mono-, di- oder trisubstituiert und die Substituenten gleich oder verschieden sein können, und

$R_7$ und $R_8$, die gleich oder verschieden sein können, Wasserstoffatome oder Alkylgruppen mit 1 bis 3 Kohlenstoffatomen bedeuten, deren Enantiomere, deren Diastereomere und deren Additionssalze.

**2.** [2.2]Paracyclophane der allgemeinen Formel

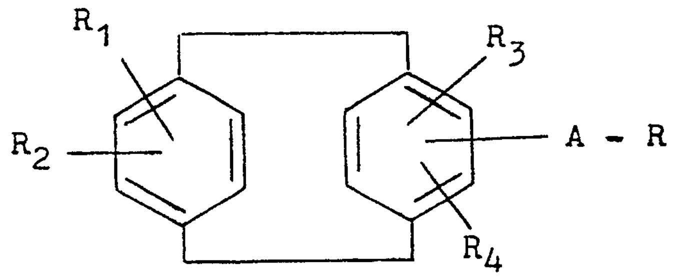 ,(Ia)

in der
A eine geradkettige Alkylengruppe mit 1 bis 5 Kohlenstoffatomen, eine $-Y_1-A_1-$ oder $-Y_2-A_2-$Gruppe, in denen
    $Y_1$ ein Sauerstoffatom und
    $A_1$ eine geradkettige Alkylengruppe mit 2 oder 3 Kohlenstoffatomen,
    $Y_2$ eine Ethenylen- oder Ethinylengruppe und
    $A_2$ eine Methylengruppe bedeuten,
wobei die Reste $A_1$ und $A_2$ mit dem Rest R verknüpft sind,

einer der Reste $R_1$, $R_2$, $R_3$ oder $R_4$ ein Wasserstoff-, Fluor-, Chlor- oder Bromatom, eine Methyl-, Hydroxy-, Methoxy- oder Methansulfonyloxygruppe und
die übrigen der Reste $R_1$, $R_2$, $R_3$ oder $R_4$ jeweils ein Wasserstoffatom oder eine Methylgruppe, und

R eine Cyanogruppe oder eine Gruppe der Formeln

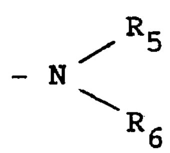 oder 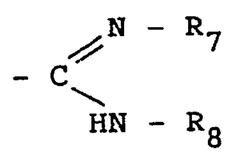

darstellen, in denen
$R_5$ ein Wasserstoffatom, eine gegebenenfalls durch eine Phenyl- oder Dimethoxyphenylgruppe substituierte Alkylgruppe mit 1 bis 3 Kohlenstoffatomen, eine Alkylgruppe mit 4 bis 8 Kohlenstoffatomen, eine Cycloalkylgruppe mit 5 bis 7 Kohlenstoffatomen oder eine bi- oder tricyclische Alkylgruppe mit 7 bis 12 Kohlenstoffatomen, wobei jeder der vorstehend erwähnten cyclischen Reste durch eine oder zwei Methylgruppen substituiert sein kann,

$R_6$ ein Wasserstoffatom oder eine Alkylgruppe mit 1 bis 3 Kohlenstoffatomen oder

$R_5$ und $R_6$ zusammen mit dem dazwischenliegenden Stickstoffatom eine Pyrrolidino-, Piperidino-, Hexamethylenimino- oder Morpholinogruppe, wobei
    i) in einer vorstehend bei der Definition der Reste $R_5$ und $R_6$ erwähnten Piperidino- oder Hexamethyleniminogruppe eine Methylengruppe in 4-Stellung durch eine Imino-, Methylimino-, Ethoxycarbonylimino-, Acetylimino-, Phenylethylimino-, Phenoxyethylimino-oder Phenylallyliminogruppe ersetzt sein kann oder
    ii) eine vorstehend bei der Definition der Reste $R_5$ und $R_6$ erwähnte Piperidinogruppe durch eine Carboxy-, Ethoxycarbonyl-, Aminocarbonyl-, Methylaminocarbonyl- oder Dimethylaminocarbonylgruppe oder durch eine durch eine Hydroxy-, Acetoxy-, Cyano-, Carboxy-, Ethoxycarbonyl-, Aminocarbonyl-, Methylaminocarbonyl-, Dimethylaminocarbonyl-, Amino-, Methylamino-, Dimethylamino-, Diethylamino-, Acetylamino-, N-Acetyl-methylamino-, Methansulfonylamino- oder N-Methansulfonyl-methylaminogruppe substituierte Methylgruppe oder durch eine in 2-Stellung durch eine Hydroxy-, Carboxy- oder Ethoxycarbonylgruppe substituierte Ethylgruppe oder durch eine in 2-, 3- oder 4-Stellung durch eine Dimethylaminocarbonylgruppe substituierte Alkylgruppe mit 2 bis 4 Kohlenstoffatomen substituiert sein kann, wobei, soweit nichts anderes erwähnt wurde, die unter i)

und ii) vorstehend erwähnten Phenylkerne jeweils durch eine Nitro- oder Trifluormethylgruppe monosubstituiert oder durch Methyl- oder Methoxygruppen mono-, di- oder trisubstituiert und die Substituenten gleich oder verschieden sein können, und

$R_7$ und $R_8$, die gleich oder verschieden sein können, Wasserstoffatome oder Methylgruppen bedeuten, deren Enantiomere, deren Diastereomere und deren Additionssalze.

**3.** Cyclophane der allgemeinen Formel Ia gemäß Anspruch 2, in der

A eine geradkettige Alkylengruppe mit 2 bis 4 Kohlenstoffatomen, eine $-Y_1-A_1-$ oder $-Y_2-A_2-$Gruppe, in denen
$Y_1$ ein Sauerstoffatom und
$A_1$ eine geradkettige Alkylengruppe mit 2 oder 3 Kohlenstoffatomen,
$Y_2$ eine Ethenylen- oder Ethinylengruppe und
$A_2$ eine Methylengruppe bedeuten,
wobei die Reste $A_1$ und $A_2$ mit dem Rest R verknüpft sind,

$R_1$, $R_2$, $R_3$ und $R_4$ jeweils ein Wasserstoffatom und

R eine Gruppe der Formeln

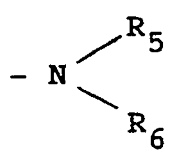

oder

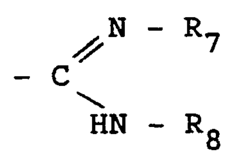

darstellen, in denen
$R_5$ ein Wasserstoffatom, eine gegebenenfalls durch eine Phenyl- oder Dimethoxyphenylgruppe substituierte Alkylgruppe mit 1 bis 3 Kohlenstoffatomen, eine Alkylgruppe mit 4 bis 8 Kohlenstoffatomen, eine Cycloalkylgruppe mit 5 bis 7 Kohlenstoffatomen oder eine bi- oder tricyclische Alkylgruppe mit 7 bis 12 Kohlenstoffatomen, wobei jeder der vorstehend erwähnten cyclischen Reste durch eine oder zwei Methylgruppen substituiert sein kann,

$R_6$ ein Wasserstoffatom oder eine Alkylgruppe mit 1 bis 3 Kohlenstoffatomen oder

$R_5$ und $R_6$ zusammen mit dem dazwischenliegenden Stickstoffatom eine Piperidinogruppe, wobei
i) in einer vorstehend bei der Definition der Reste $R_5$ und $R_6$ erwähnten Piperidinogruppe eine Methylengruppe in 4-Stellung durch eine Imino-, Methylimino-, Ethoxycarbonylimino-, Acetylimino-, Phenylethylimino-, Phenoxyethylimino- oder Phenylallyliminogruppe ersetzt sein kann oder
ii) eine vorstehend bei der Definition der Reste $R_5$ und $R_6$ erwähnte Piperidinogruppe in 3- oder 4-Stellung durch eine Carboxy-, Ethoxycarbonyl-, Aminocarbonyl-, Methylaminocarbonyl-oder Dimethylaminocarbonylgruppe oder durch eine durch eine Hydroxy-, Acetoxy-, Cyano-, Carboxy-, Ethoxycarbonyl-, Aminocarbonyl-, Methylaminocarbonyl-, Dimethylaminocarbonyl-, Amino-, Methylamino-, Dimethylamino-, Diethylamino-, Acetylamino-, N-Acetyl-methylamino-, Methansulfonylamino- oder N-Methansulfonyl-methylaminogruppe substituierte Methylgruppe oder durch eine in 2-Stellung durch eine Hydroxy-, Carboxy- oder Ethoxycarbonylgruppe substituierte Ethylgruppe oder durch eine in 2-, 3- oder 4-Stellung durch eine Dimethylaminocarbonylgruppe substituierte Alkylgruppe mit 2 bis 4 Kohlenstoffatomen substituiert sein kann, wobei, soweit nichts anderes erwähnt wurde, die unter i) und ii) vorstehend erwähnten Phenylkerne jeweils durch eine Methyl-, Nitro- oder Trifluormethylgruppe monosubstituiert oder durch Methoxygruppen mono-, di- oder trisubstituiert sein können, und

$R_7$ und $R_8$ jeweils ein Wasserstoffatom oder eine Methylgruppe bedeuten, deren Enantiomere, deren Diastereomere und deren Additionssalze.

**4.** Folgende Cyclophane der allgemeinen Formel Ia gemäß Anspruch 2:

4-(3-(Piperidin-1-yl)prop-1-yl)-[2.2]-paracyclophan,

4-(3-(3-(N-Acetyl-methylaminomethyl)piperidin-1-yl)prop-1-yl)-[2.2]paracyclophan,

4-Methyl-7-(3-(3-(N-acetyl-methylaminomethyl)-piperidin-1-yl)-prop-l-yl)[2.2]paracyclophan,

4-(3-(3-(3-Dimethylamino-3-oxo-prop-1-yl)piperidin-1-yl)-prop-1-yl)-[2.2]paracyclophan und

4-(2-(Piperidin-1-yl)ethoxy-[2.2]paracyclophan, deren Enantiomere, deren Diastereomere und deren Additionssalze.

5. Physiologisch verträgliche Additionssalze der Verbindungen nach mindestens einem der Ansprüche 1 bis 4 mit anorganischen oder organischen Säuren.

6. Arzneimittel, enthaltend eine Verbindung nach mindestens einem der Ansprüche 1 bis 4 oder ein physiologisch verträgliches Additionssalz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 5 zur Herstellung eines Arzneimittels, das zur Behandlung der Hypertonie sowie zur Prophylaxe und Therapie ischämischer Herzerkrankungen geeignet ist.

8. Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

9. Verfahren zur Herstellung der Cyclophane der allgemeinen Formel I gemäß den Ansprüchen 1 bis 5, dadurch gekennzeichnet, daß

a) eine Verbindung der allgemeinen Formel

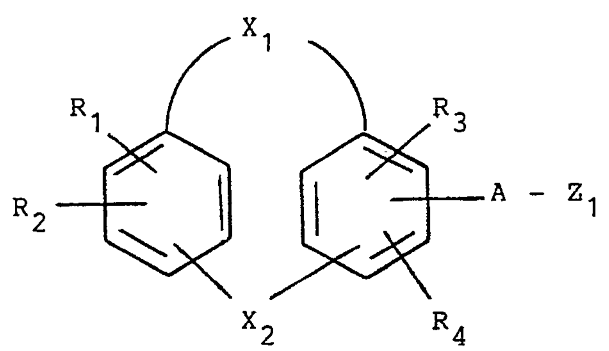

, (II)

in der
$R_1$ bis $R_4$, $X_1$, $X_2$ und A wie mindestens in einem der Ansprüche 1 bis 4 definiert sind und

$Z_1$ eine nukleophile Austrittsgruppe wie ein Halogenatom oder eine substituierte Sulfonyloxygruppe darstellt, mit einem Amin der allgemeinen Formel

H - R ,(III)

in der
R wie mindestens in einem der Ansprüche 1 bis 4 definiert ist, oder mit dessen Alkalisalz umgesetzt und, zur Herstellung einer entsprechenden Amidinoverbindung der allgemeinen Formel I, anschließend eine so erhaltene Cyanoverbindung der allgemeinen Formel I über ihren Iminoester mit einem Amin der allgemeinen Formel

$R_7$ - $NH_2$ ,(IV)

in der
$R_7$ wie mindestens in einem der Ansprüche 1 bis 4 definiert ist, umgesetzt wird oder
b) zur Herstellung einer Verbindung der allgemeinen Formel I, in der R keine Cyano-, Amidino- oder Guanodinogruppe und $Y_1$ keine Sulfinyl- oder Sulfonylgruppe darstellen sowie der Rest R keine Carbonylfunktion enthält, eine Verbindung der allgemeinen Formel

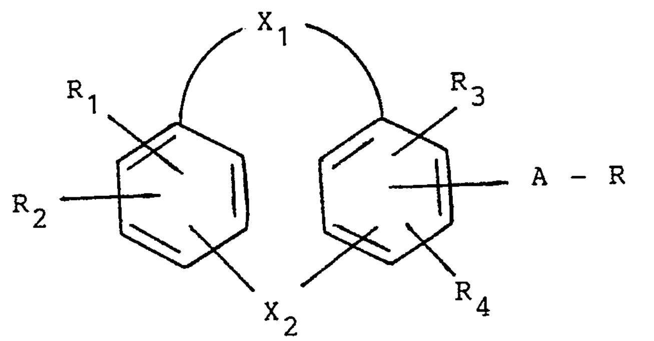
,(V)

in der
R und A wie mindestens in einem der Ansprüche 1 bis 4 definiert sind, wobei jedoch in A, $A_1$ oder $A_2$ eine zum Rest R benachbarte $-CH_2$-Gruppe durch eine -CO-Gruppe oder eine benachbarte $-CH_2-CH_2$-Gruppe durch eine -CO-CO-Gruppe ersetzt sein muß, mit einem komplexen Metallhydrid reduziert wird

und erforderlichenfalls anschließend ein während den Umsetzungen a) und b) zum Schutze von reaktiven Gruppen wie Hydroxy-, Amino-, Imino- oder Alkylaminogruppen verwendeter Schutzrest abgespalten wird und/oder

gewünschtenfalls eine so erhaltene Verbindung der allgemeinen Formel I, in der R eine Alkoxycarbonylgruppe enthält, mittels Hydrolyse in eine entsprechende Hydroxycarbonylverbindung der allgemeinen Formel I übergeführt wird, wobei gleichzeitig eine im Reaktionsgemisch entstehende Hydroxycarbonylimino- oder Hydroxycarbonylaminoverbindung der allgemeinen Formel I beim Erhitzen zu der entsprechenden Imino- oder Aminoverbindung der allgemeinen Formel I decarboxyliert wird, oder

eine so erhaltene Verbindung der allgemeinen Formel I, in der R eine Aminocarbonylgruppe enthält, mittels Dehydratisierung in eine entsprechende Cyanoverbindung der allgemeinen Formel I übergeführt wird oder

eine so erhaltene Verbindung der allgemeinen Formel I, in der R eine Imino-, Amino- oder Alkylaminogruppe enthält, mittels Acylierung in eine entsprechende Alkanoylimino-, Alkanoylamino-, N-Alkanoyl-alkylamino-, Alkylsulfonylimino-, Alkylsulfonylamino-oder N-Alkylsulfonyl-alkylaminoverbindung der allgemeinen Formel I übergeführt wird oder

eine so erhaltene Verbindung der allgemeinen Formel I, in der $R_5$ und $R_6$ jeweils ein Wasserstoffatom darstellen, mittels

Umamidinierung in eine entsprechende Amidinoverbindung der allgemeinen Formel I übergeführt wird oder

eine so erhaltene Verbindung der Formel I in ihre Diastereomeren und in ihre Enantiomeren aufgetrennt wird und/oder

eine so erhaltene Verbindung der Formel I in ihre Säureadditionssalze, insbesondere für die pharmazeutische Anwendung in ihre physiologisch verträglichen Salze mit anorganischen oder

organischen Säuren, übergeführt wird.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 91 10 4535

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEM. BER., Band 120, 1987, Seiten 1825-1828, VCH Verlaggesellschaft mbH, Weinheim, DE; M. PSIORZ et al.: "Ein einfacher Zugang zu funktionalisierten [2.2]paracyclophanen"<br>* Whole article *<br>----- | 1-9 | C 07 D 211/26<br>C 07 D 211/34<br>C 07 D 295/03<br>C 07 D 295/185<br>C 07 D 295/088<br>C 07 D 211/60<br>C 07 D 243/08<br>C 07 D 211/22<br>C 07 D 211/62<br>C 07 C 211/31<br>C 07 C 257/14<br>C 07 C 279/04<br>A 61 K 31/53<br>A 61 K 31/445<br>C 07 D 295/096 |

RECHERCHIERTE SACHGEBIETE (Int. Cl.4)

C 07 D 211/00
C 07 D 295/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 2-4
Unvollständig recherchierte Patentansprüche:
Nicht recherchierte Patentansprüche: 1,5-9
Grund für die Beschränkung der Recherche:

Ansprüche 1 und 5-9 sind zu weit gefasst und verstossen gegen Art. 84 EPC ("die Ansprüche müssen von der Beschreibung gestütz sein"). Es wurden lediglich (2.2.)-p-cyclophane recherchiert.

| Recherchenort | Abschlußdatum der Recherche | Prüfer |
|---|---|---|
| DEN HAAG | 02-07-1991 | KISSLER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument